# EUROPEAN PATENT APPLICATION

(11) **EP 1 346 985 A1**
(43) Date of publication of application: **24.09.2003**
(21) Application number: 01995000.5
(22) Date of filing: 25.12.2001
(51) Int. Cl.: C07D 239/47, C07D 413/12, C07D 239/54, C07D 405/04, A61K 31/4245

(54) **PYRIMIDINE DERIVATIVES AND PROCESS FOR PREPARING THE SAME**

(30) Priority: 26.12.2000 JP 2000395012
(71) Applicant: ONO PHARMACEUTICAL CO., LTD., Osaka-shi, Osaka 541-8526 (JP)
(72) Inventor: SUGIURA, Tsuneyuki, Mishima-gun, Osaka 618-8585 (JP); HORIUCHI, Toshihide, Mishima-gun, Osaka 618-8585 (JP); MIYAZAKI, Toru, Mishima-gun, Osaka 618-8585 (JP); KOJIMA, Tsutomu, Sakai-gun, Fukui 913 (JP); HASHIMOTO, Shinsuke, Sakai-gun, Fukui 913 (JP)
(74) Representative: Henkel, Feiler, Hänzel
(86) International application number: JP0111371
(87) International publication number: WO02051815

(57) **Abstract**

A pyrimidine derivative compound of formula (I) or a salt thereof and a method for the preparation thereof.

The pyrimidine derivative of formula (I) or a salt thereof is useful as important synthetic intermediate for a pharmaceutical a drug. The method for the preparation of the present invention can provide the compound of formula (E), which is useful as a pharmaceutical drug, more efficiently than conventional method does.

(All symbols in the formulae have the same meaning as depicted in the specification)

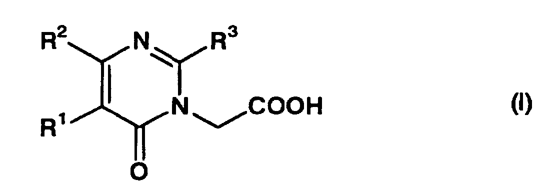

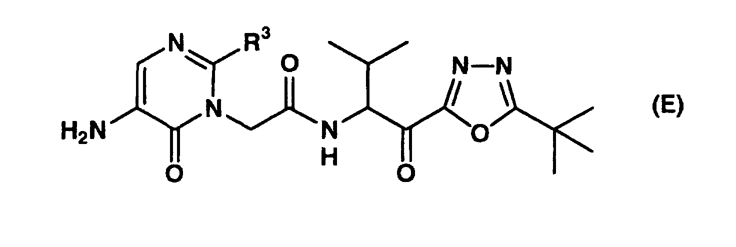

## Description

### Technical Field

The present invention relates to a novel pyrimidine derivative of formula (I), which is useful as an intermediate for a pharmaceutical drug and/or a method for the preparation of a pharmaceutical drug utilizing it.

More specifically, the present invention relates to
(1) a pyrimidine derivative of formula (I) 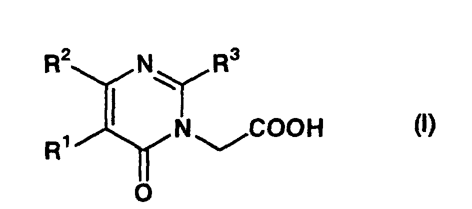 (wherein all symbols have the same meaning as below depicted),
(2) a method for the preparation thereof and
(3) a method for the preparation of (RS)-N-[1-(5-tert-butyl-1,3,4-oxadiazol-2-ylcarbonyl)-2-methylpropyl]-2-(5-amino-6-oxo-1,6-dihydropyrimidin-1-yl)acetamide derivative compound of formula (E) 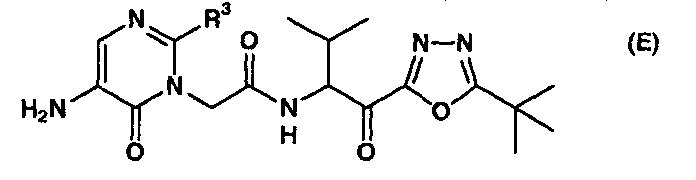 utilizing it or a non-toxic salt thereof.

### Background

W098/24806 discloses that the compound of formula (E) or a non-toxic salt thereof is useful as an inhibitor of serine proteases (particularly elastase).

J. Med. Chem., vol. 43, 4927-4929(2000) discloses that, among the compound of formula (E), the compound wherein R³ is phenyl, i.e. (RS)-N-[1-(5-tert-butyl-1,3,4-oxadiazol-2-ylcarbonyl)-2-methylpropyl]-2-(5-amino-6-oxo-1,6-dihydropyrimidin-1-yl)acetamide of formula (E-1) 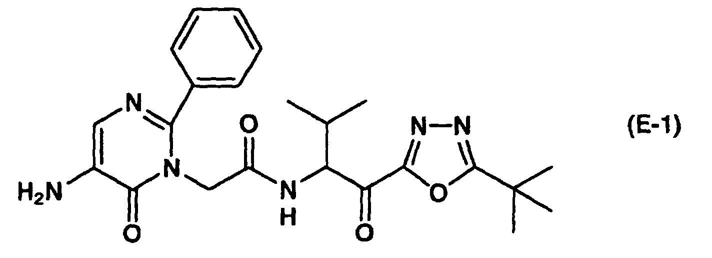 or a non-toxic salt thereof is useful as an orally active elastase inhibitor.

Hence the method for the preparation of the compound of formula (E-1) has been investigated in various way. By now the following methods have been known.
(1) WO98/2480 discloses a method for the preparation of the compound of formula (E-1), according to the method described in J. Med. Chem., vol.39, 98-108 (1995) (shown in reaction scheme 1) to prepare 5-benzyloxycarbonylamino-6-oxo-2-phenyl-1,6-dihydropyrimidin-1-ylacetic acid of formula (V-1), followed by subjecting the key intermediate (V-1) to the method shown in reaction scheme 2. 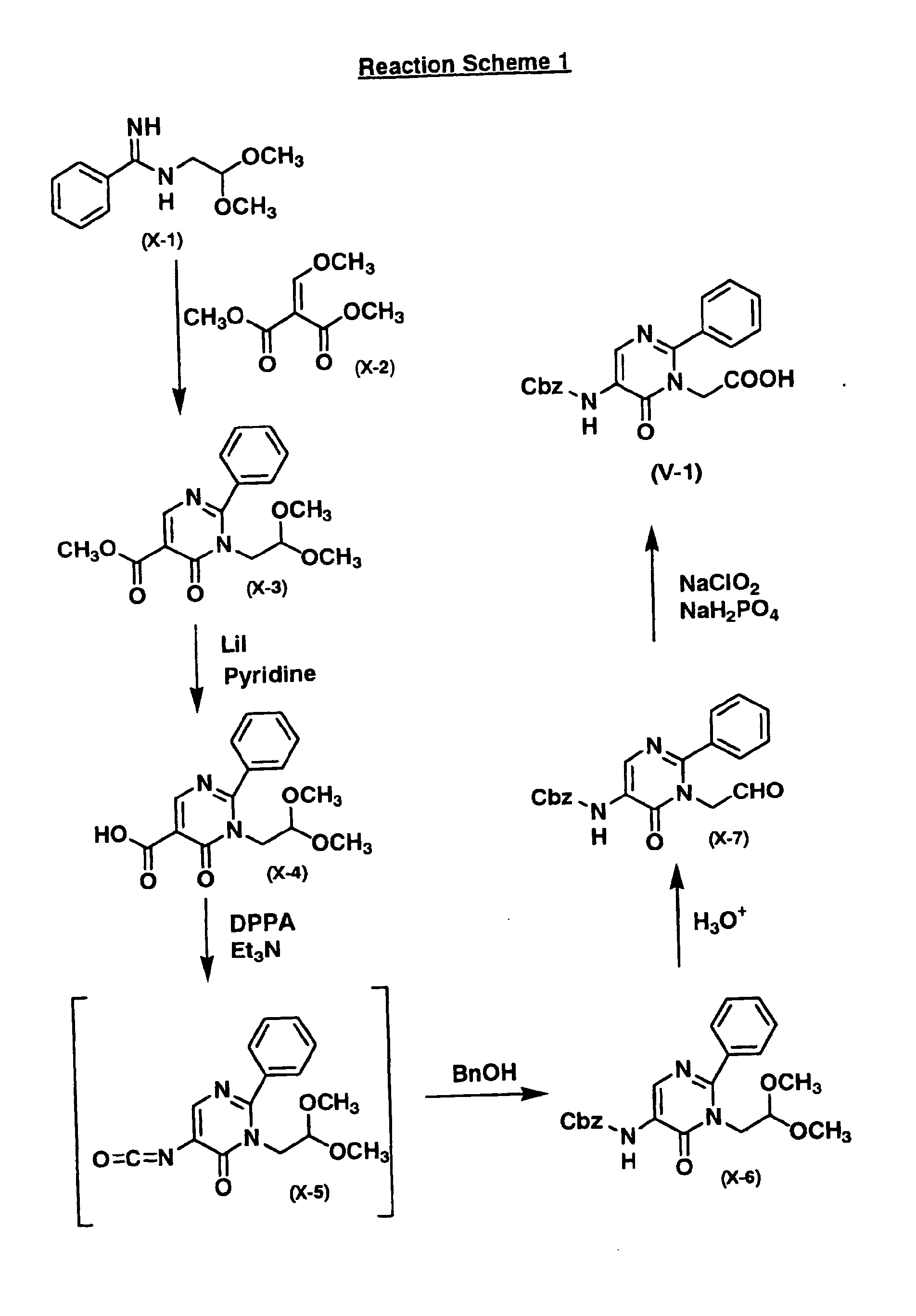 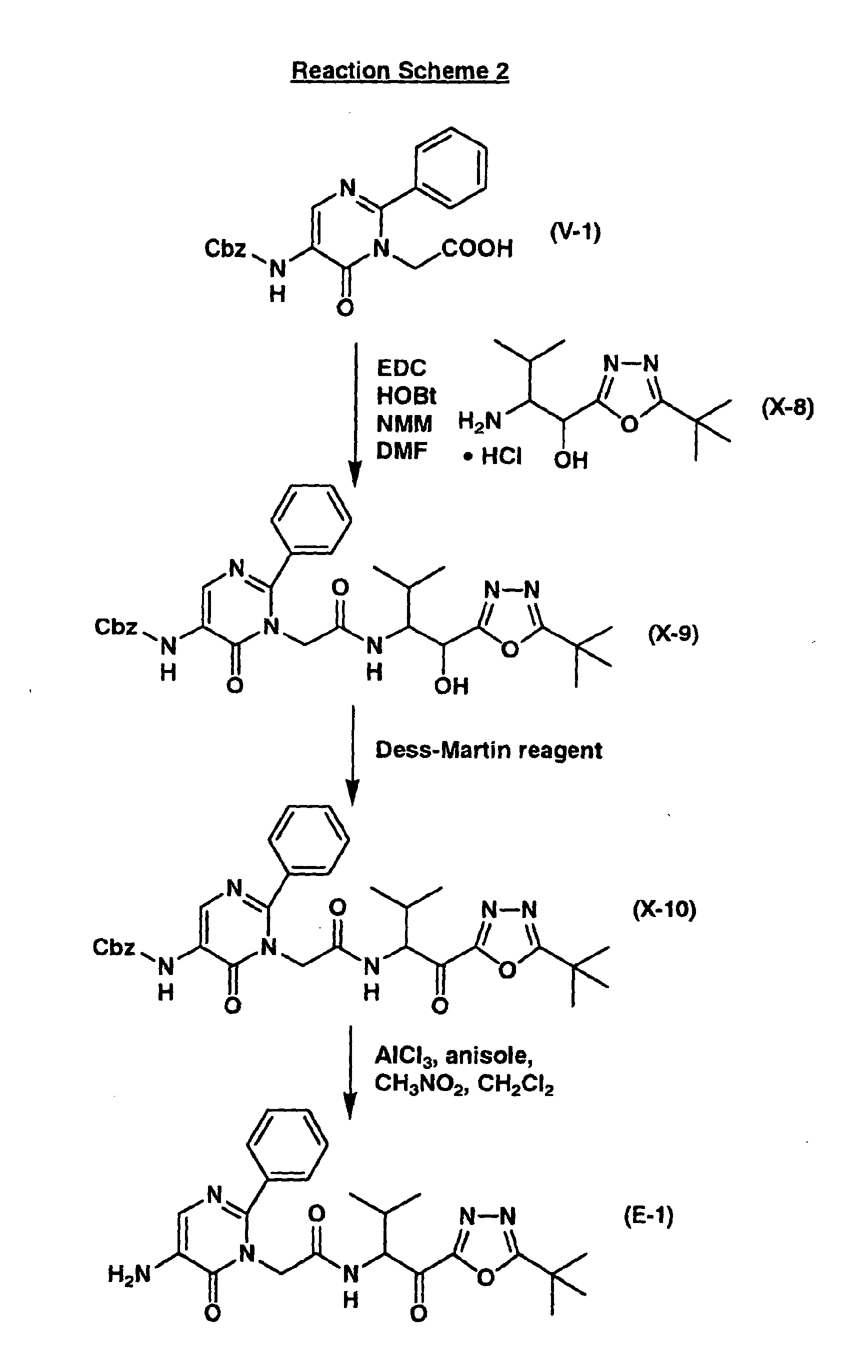
   In reaction scheme 1 and 2, Et₃N is triethylamine, DPPA is diphenylphosphorylazide, BnOH is benzyl alcohol, Cbz is benzyloxycarbonyl, EDC is 1 -ethyl-3-(3-dimethylamino propyl)carbodiimide, HOBt is 1-hydroxybenzotriazole, NMM is N-methylmorpholine, and DMF is dimethylformamide.
   The method of reaction scheme 1 discloses Curtius rearrangement reaction in order to prepare the compound of formula (X-6) from the compound of formula (X-4). However, this rearrangement reaction generates nitrogen gas to a great degree in the course of reaction. This nitrogen gas does not matter in a small-scale synthesis such as in laboratory level, but in the industrial mass synthesis it matters significantly. From this aspect, the method for the preparation of a pyrimidine derivative that does not go through Curtius rearrangement reaction has been hoped for.
   And the method shown in W098/24806 the pyrimidine derivative of formula (E-1) is prepared in 8 steps, starting from a reaction of an amidine compound of formula (X-1) and dimethyl methoxymethylene malonate of formula (X-2). Therefore, in the mass synthesis of pyrimidine derivative of formula (E-1) more efficient method that gives a short-way method has been hoped for.
(2) WO0O/551 discloses an improved method for the preparation of the compound of formula (X-6) from the compound of formula (X-4) in reaction scheme 1. That is to say, this method gives a method for the preparation of pyrimidine derivative, which is not mediated by Curtius rearrangement. 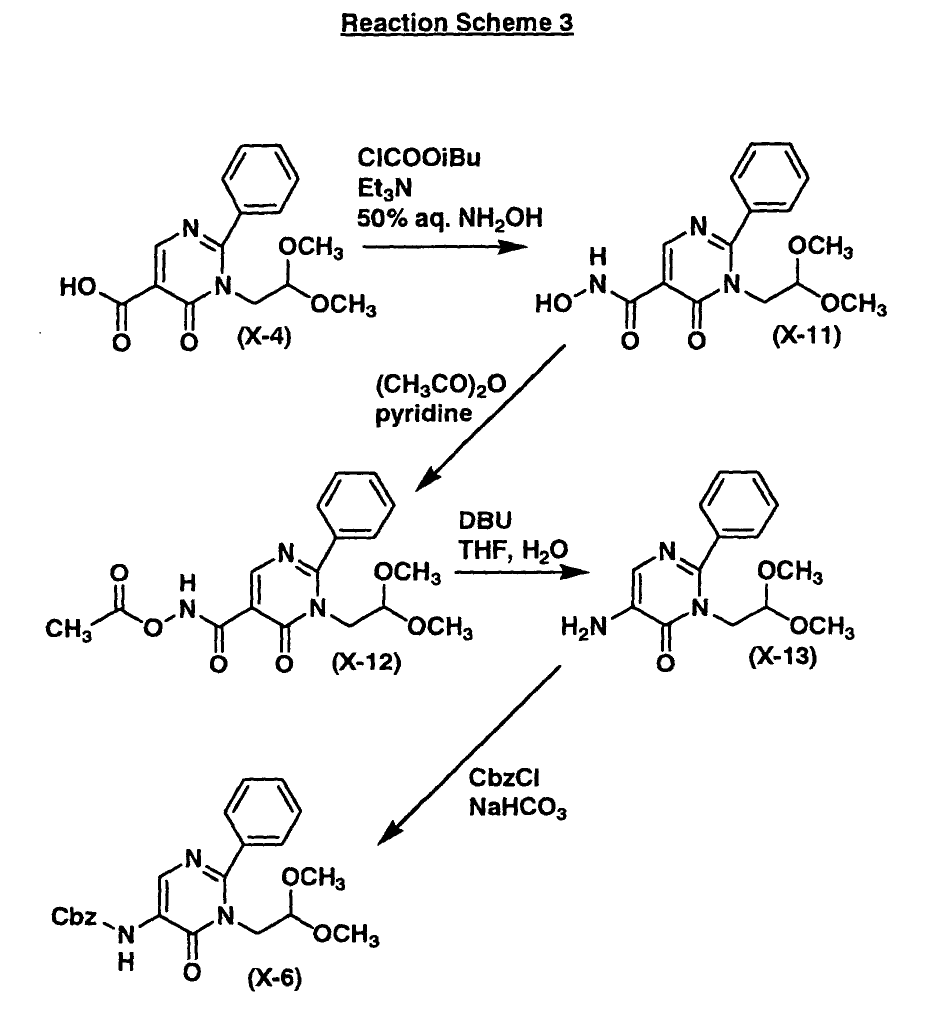
   In reaction scheme 3, Et₃N is triethylamine, ClCOOiBU is isobutyl chlorocarbonate, DBU is 1,8-diazabicyclo [5.4.0]-7-undecene, THF is tetrahydrofuran, and CbzCl is benzyloxycarbonyl chloride.
   The method of reaction scheme 3 can avoid Curtius rearrangement reaction, but in order to prepare the compound of formula (X-6) it requires 4 steps from the compound of formula (X-4). Therefore, for the industrial mass synthesis, more efficient method, which requires only fewer steps, has been hoped for.
(3) WO00/55145 discloses a method for the preparation of (RS)-N-[1-(5-tert-butyl-1,3,4-oxadiazol-2-ylcarbonyl)-2-methylpropyl ] -2-(5-amino-6-oxo-2-phenyl-1,6-dihydropyrimidine-l-yl)acetamide of formula (E-1) or a non-toxic salt thereof (see reaction scheme 4).

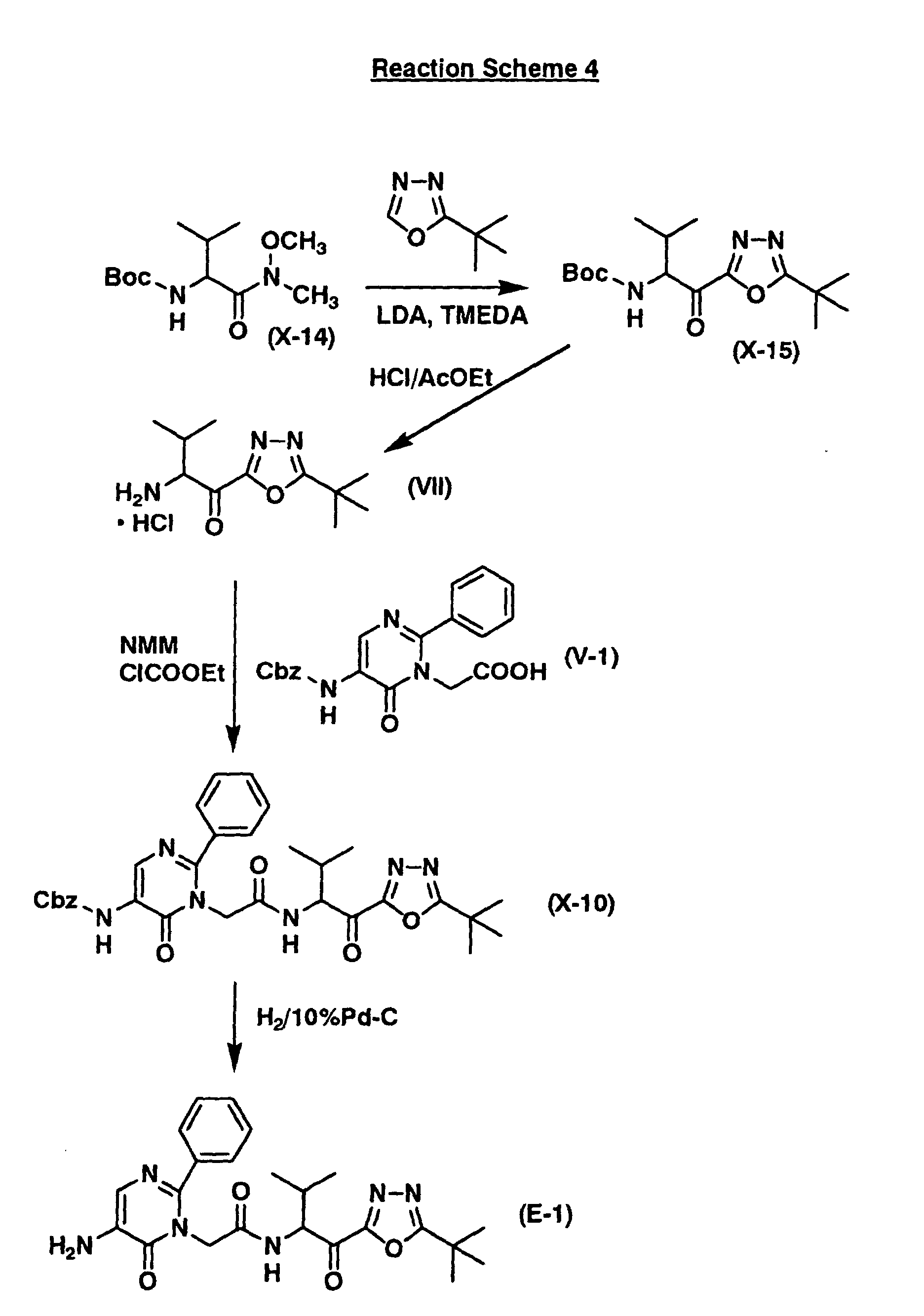

In reaction scheme 4, LDA is lithium diisopropylamide, TMEDA is N,N,N',N'-tetramethylethylenediamine, Boc is tert-butyloxycarbonyl, AcOEt is ethyl acetate, NMM is N-methyl morpholine, C1COOEt is ethyl chlorocarbonate and Cbz is benzyloxycarbonyl.

The method of reaction scheme 4 require deprotection reaction of benzyloxycarbonyl after subjecting to amidation reaction the compound of formula (V-1) or a salt thereof and the compound of formula (VII) or a salt thereof. In addition, the deprotection reaction has a disadvantage in industrialization that it must be carried out in a very dilute solution. Therefore, a more efficient method, which requires fewer processes, has been desired.

### Disclosure of the invention

The present inventors have energetically investigated for the purpose of preparing the compound of formula (E) efficiently in an industrial mass production scale, to find out that the purpose was accomplished by way of the compound of formula (I-A).

That is, the present inventors succeeded in preparing the compound of formula (E) without deprotection reaction by subjecting to amidation reaction 5-amino-6-oxo-1,6-dihydropyrimidin-1-ylacetic acid derivative of formula (I-A) 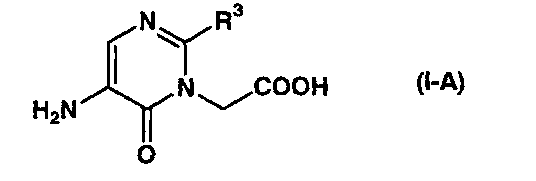 (wherein R³ has the same meaning as above depicted) or a salt thereof and (RS)-2-(2-amino-3-methylbutyryl)-5-tert-butyl-1,3,4-oxadizaole of formula (VII) 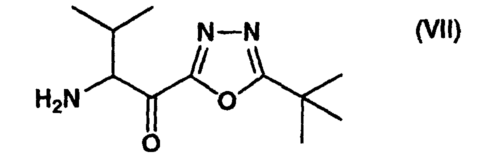 or a salt thereof.

5-amino-6-oxo-1,6-dihydropyrimidin-1-ylacetic acid derivative compound of formula (I-A) or a salt thereof is a novel compound which is not known so far.

The present method has made it possible to prepare the compound of formula (E-1), which is important as a pharmaceutical drug efficiently.

The present inventors have further found a new method for the preparation of the compound of formula (I-A).

That is,
(1) the present inventors have succeeded in preparing the compound of formula (I-A) or a salt thereof by giving 5-benzoylamino-6-oxo-1,6-dihydro pyrimidin-1-ylacetic acid derivative of formula (I-B) 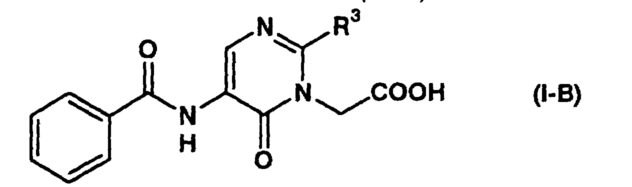 or a salt thereof by subjecting to a reaction 4-ethoxymethylen-2-phenyloxazolin-5-one of formula (II-1) or 4-methoxymethylen-2-phenyloxazolin-5-one of formula (II-2) and 4-ethoxymethylen-2-phenyloxazolin-5-one and an amidinoacetic acid derivative of formula(III) 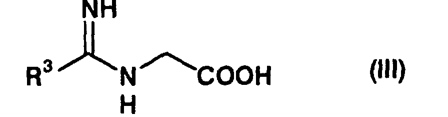 (wherein R³ has the same meaning as above depicted) or a salt thereof.
   The series of the reactions do not go through Curtius rearrangement reaction. And the compound of formula (E) is prepared in 2-3 steps from the compound of formula (II-1) or the compound of formula (II-2) and the compound of formula (III). Therefore, this method is very efficient in industrial mass synthesis.
   Additionally, 5-benzoylamino-6-oxo-2-1,6-dihydro pyrimidin-1-ylacetic acid derivative of formula (I-B) or a salt thereof is novel, which has not been known at all so far.
(2) Furthermore, the present inventors have succeeded in preparing the compound of formula (I-A), by subjecting to a reaction dimethyl benzyloxycarbonylaminomalonate of formula (IV-1) 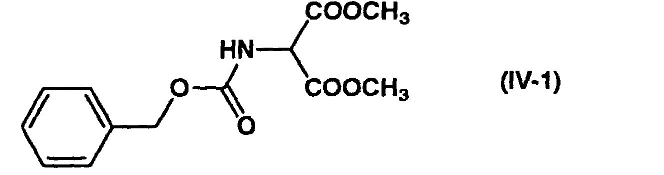 or dimethyl benzyloxycarbonylaminomalonate of formula (IV-2) 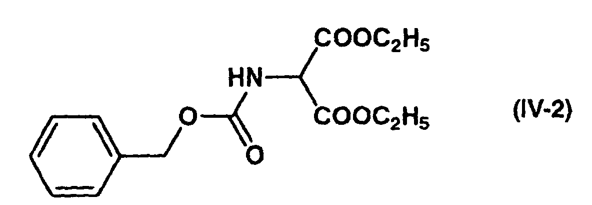 and amidinoacetic acid derivative of formula (III) 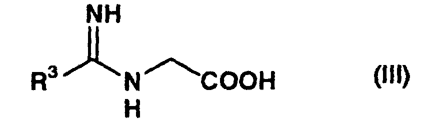 (wherein R³ has the same meaning as below described) or a salt thereof, to give 4-hydroxy-5-benzyloxycarbonylamino-6-oxo-1,6-dihydropyrimidin-1-ylacetic acid derivative of formula (I-C) 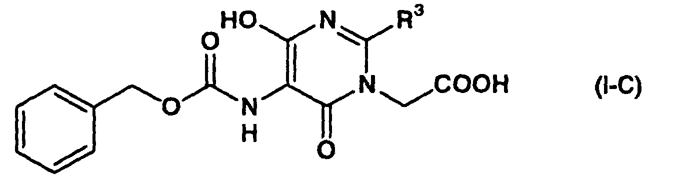 (wherein R³ has the same meaning as below described) or a salt thereof, followed by subjecting to a halogenation reaction to give 4-chloro-5-benzyloxycarbonylamino-6-oxo-1,6-dihydropyrimidin-1-ylacetic acid derivative of formula (I-D) 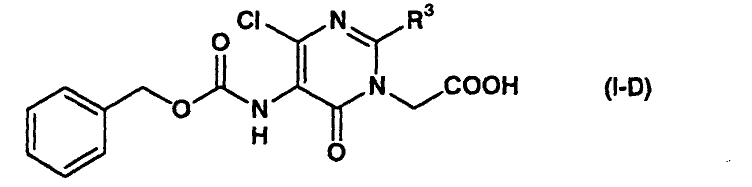 (wherein R³ has the same meaning as below described) or a salt thereof, and followed by subjecting to hydrogenation reaction.
   The series of reactions do not go through Curtius rearrangement reaction, either. And the compound of formula (E) is prepared in a short step (i.e. 4 steps) from the compound of formula (IV-1) or the compound of formula (IV-2) and the compound of formula (III). Therefore, this method is also very efficient in industrial mass synthesis.
   Moreover, 4-hydroxy-5-benzyloxycarbonylamino-6-oxo-1,6-dihydropyrimidin-1-ylacetic acid derivative of formula (I-C) or a salt thereof and 4-chloro-5-benzyloxycarbonylamino-6-oxo-1,6-dihydropyrimimidin-1-ylacetic acid or a salt thereof is novel, which has not been known at all so far.
(3) The present inventors also have succeeded in preparing the compound of formula (I-A) by subjecting to deprotection reaction 5-benzyloxycarbonylamino-6-oxo-1,6-dihydro pyrimidin-l-vlacetic acid derivative of formula (V) 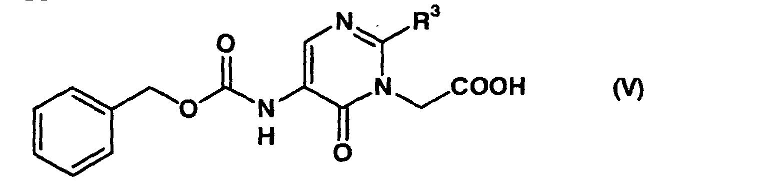 (wherein R³ has the same meaning as below).
(4) The present inventors have succeeded in preparing 5-nitro-6-oxo-1,6-dihydropyrimidin-1-ylacetic acid derivative of formula (I-A) by subjecting to a reaction the compound of (VI) 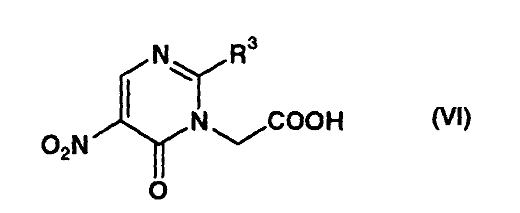 (wherein R³ has the same meaning as below described).

Therefore, the pyrimidine derivative of formula (I) of the present invention or a salt thereof is utterly novel and these compounds are useful as intermediates for (RS)-N-[1-(5-tert-butyl-1,3,4-oxadiazol-2-ylcarbonyl)-2-methylpropyl]-2-(5-amino-6-oxo-1,6-dihydropyrimidin-1-yl)acetamide derivative or a non-toxic salt thereof (W098/24806).

### Description of the invention

The present invention relates to
1) a pyrimidine derivative of formula (I) 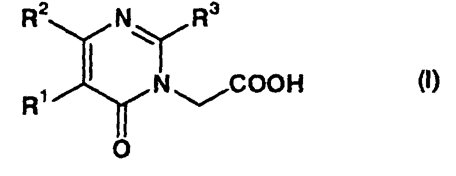 (wherein R¹ is amino, benzoylamino or benzyloxycarbonylamino,
   R² is hydrogen, hydroxy or chlorine,
   R³ is
   (1) C1~4 alkyl, (2) Cyc1 or (3) C1-4 alkyl substituted with Cyc1
   (wherein Cyc 1 is C3~10 mono- or bi-cyclic carboring or 3~10 membered mono- or bi-cyclic heteroring comprising 1-4 of nitrogen, 1-2 of oxygen and/or 1-2 of sulfur and Cyc1 may be substituted with 1-5 of R⁴, R⁴ is
   (1) C1~4 alkyl, (2) halogen, (3) nitro, (4) trifluoromethyl, (5) trifluoromethoxy, (6) nitrile, (7) phenyl or (8) -OR⁵ (wherein R⁵ is C1-4 alkyl, phenyl, C1-4 alkyl substituted with phenyl)),
   with proviso that when R¹ is benzyloxycarbonylamino, R² is hydroxy or chlorine) or a salt thereof,
2) a method for the preparation thereof and
3) a method for the preparation of (RS)-N-[1-(5-tert-butyl-1,3,4-oxadiazol-2-ylcxarbonyl)-2-methylpropyl]-2-(5-amino-6-oxo-1,6-dihydropyrimidin-1-yl)acetamide derivative of formula (E) 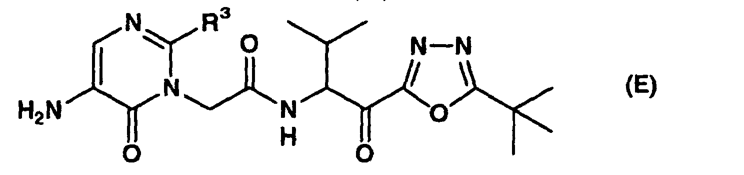 (wherein R³ has the same meaning as depicted in above 1)) or a salt thereof, characterized by utilizing it.

In the present specification, C1-4 alkyl is methyl, ethyl, propyl, butyl and isomers thereof.

In the present specification, halogen is fluorine, chlorine, bromine and iodine.

In the present specification, C3-10 mono- or bi-cyclic carboring is C3-10 mono- or bi-cyclic carboaryl or partially or completely saturated one thereof. For example, cyclopropane, cyclobutane, cyclopentane, cyclohexane, cycloheptane, cyclooctane, cyclononane, cyclodecane, cyclopropene, cyclobutene, cyclopentene, cyclohexene, cycloheptene, cyclooctene, cyclopentadiene, cyclohexadiene, cycloheptadiene, cyclooctadiene, benzene, pentalene, azulene, perhydroazulene, perhydropentalene, indene, perhydroindene, indan, naphthalene, tetrahydronaphthalene, perhydronaphthalene, etc. are included.

In the present specification, 3-10 membered mono- or bi-cyclic heteroring comprising 1-4 of nitrogen, 1-2 of oxygen and/or 1-2 of sulfur is 3-10 membered mono- or bi-cyclic heteroaryl comprising 1-4 of nitrogen, 1-2 of oxygen and/or 1-2 of sulfur or partially or completely saturated one thereof.

3-10 membered mono- or bi-cyclic heteroaryl comprising 1-4 of nitrogen, 1-2 of oxygen and/or 1-2 of sulfur is, for example, pyrrole, imidazole, triazole, tetrazole, pyrazole, pyridine, pyrazine, pyrimidine, pyridazine, azepine, diazepine, furan, pyran, oxepine, thiophene, thiin, thiepine, oxazole, isoxazole, thiazole, isothiazole, furazane, oxadiazole, oxazine, oxadiazine, oxazepine, oxadiazepine, thiadiazole, thiazine, thiadiazine, thiazepine, thiadiazepine, indole, isoindole, indolidine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzoimidazole, chromene, benzofurazane, benzothiadiazole, benzotriazole, etc.

Completely or partially saturated ones of 3-10 membered mono- or bi-cyclic heteroaryl comprising 1-4 of nitrogen, 1-2 of oxygen and/or 1-2 of sulfur include, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiin (dihydrothiopyran), tetrahydrothiin (tetrahydrothiopyran), dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane, benzodithiane, etc.

Unless otherwise specified, all isomers are included in the present invention. For example, alkyl, alkenyl and alkynyl groups include straight-chain and also branched-chain ones. In addition, isomers in doublebond, ring, fused ring (E-, Z-, cis-, trans-isomer), isomers generated from asymmetric carbon atoms) (R-, S-, α-, β-isomer, enantiomer, diastereomer), optically active isomers having optical rotation (D-, L-, d-, l-isomer), polar compounds separated by chromatography (more polar compound, less polar compound), equilibrium compounds, mixtures thereof at arbitrary ratios and racemic mixtures are included in branched-chain alkyl are included in the present invention.

### [The method for the preparation of the compounds of the present invention]

The compound of formula (I) of the present invention may be prepared according to the following methods or the methods described in the examples.
[1] Among the compound of formula (I) of the present invention, the compound wherein R¹ is benzoylamino and R² is hydrogen, i.e. 5-(benzoyl)amino-6-oxo-1,6-dihydro pyrimidin-1-vlacetic acid derivative of formula (I-B) 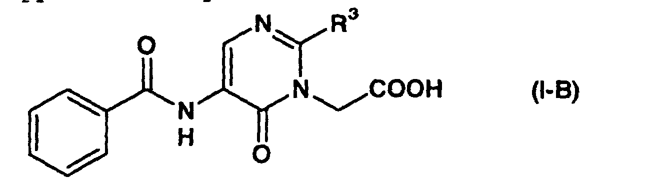 (wherein R² has the same meaning as above described) or a salt thereof may be prepared by subjecting to a reaction 4-ethoxymethylen-2-phenyloxazolin-5-one of formula (II-1) 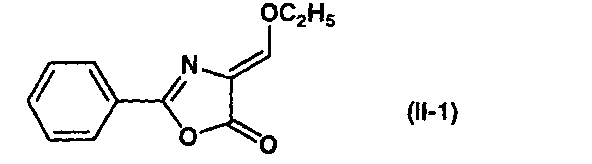 or 4-methoxymethylen-2-phenyloxazolin-5-one of formula (11-2) 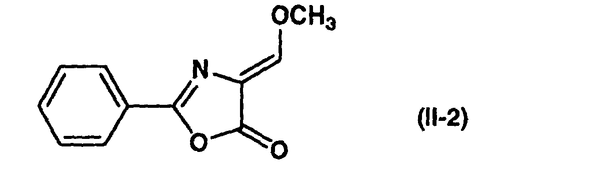 and amidinoacetic acid derivative of formula (III) 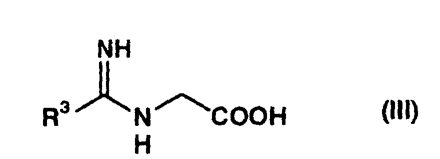 (wherein R² has the same meaning as above described) or a salt thereof.
   The reaction of the compound of formula (II-1) or the compound of formula (11-2) and the compound of formula (III) or a salt thereof is, for example, carried out in an organic solvent (methanol, ethanol, etc.) in the presence of a base (sodium ethylate, sodium methylate, etc.) at a temperature of 20-150°C.
[2] Among the compound of formula (I) of the present invention, the compound wherein R¹ is benzyloxycarbonyl and R² is hydroxy, i.e. 4-hydroxy-5-benzyloxycarbonylamino-6-oxo-1,6-dihydropyrimidin-1-ylacetic acid derivative of formula (I-C) 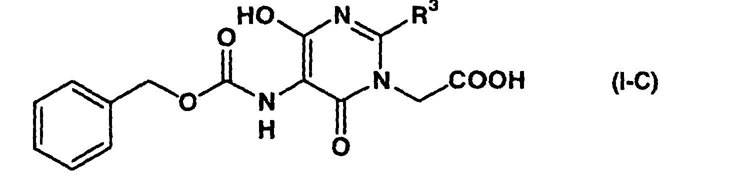 (wherein R² has the same meaning as above described) or a salt thereof may be prepared by subjecting to a reaction dimethyl benzyloxycarbonylaminomalonate of formula (IV-1) 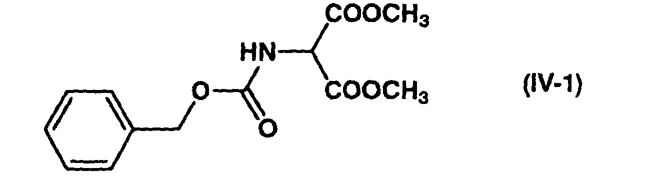 or a salt thereof or diethyl benzyloxycarbonylaminomalonate of formula (IV-2) 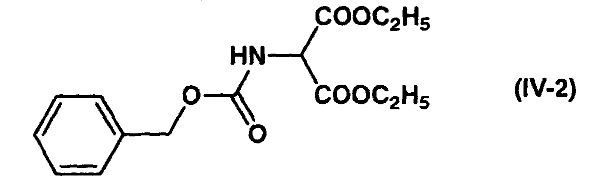 or a salt thereof and amidinoacetic acid derivative of formula (III) 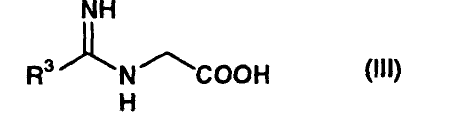 (wherein R³ has the same meaning as above described) or a salt thereof.
[3] Among the compound of formula (I) of the present invention, the compound wherein R¹ is benzyloxycarbonyl and R² is chlorine, i.e. 4-chloro-5-benzyloxycarbonyl amino-6-oxo-1,6-dihydropyridimin-1-ylacetic acid derivative of formula (I-D) 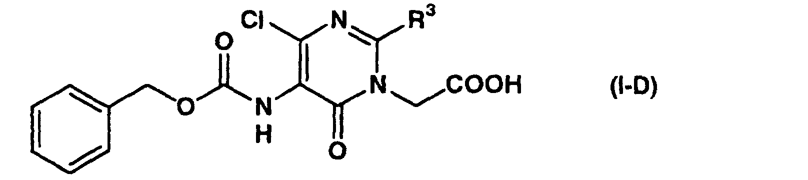 (wherein R³ has the same meaning as above described) or a salt thereof may be prepared by subjecting to a halogenation reaction the compound of formula (I-C).
   The halogenation reaction is known, for example, it is carried out in an organic solvent (dichloromethane, chloroform, etc.) in the presence of halogenating reagent (phosphorous oxychloride, thionyl chloride, etc.) at a temperature of -20-100°C.
[4] Among the compound of formula (I) of the present invention, the compound wherein R³ is hydrogen, i.e. 5-amino-6-oxo-1,6-dihydropyrimidin-1-ylacetic acid derivative of formula (I-A) 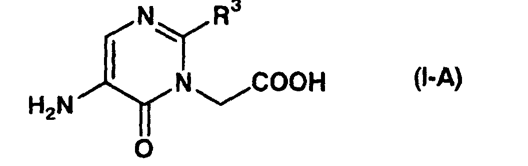 (wherein R³ has the same meaning as above described) or a salt thereof may be prepared according to the following methods of (a)-(d).
   (a) The compound of formula (I-A) or a salt thereof may be prepared by subjecting to a deprotection reaction the compound of formula (I-B), prepared according to the previous method.
      The deprotection reaction is known, for example, it is carried out in an organic solvent (methanol, ethanol, etc.) in the presence of a base (sodium methylate, sodium ethylate, etc.) at a temperature of 0~150°C.
      The compound of formula (I-A) or a salt thereof may also be prepared by subjecting to a reaction the compound of formula (II-1) or the compound of formula (11-2) and the compound of formula (III) or a salt thereof, to give the compound of formula (I-B) or a salt thereof, followed by subjecting to a deprotection reaction without isolation (i.e. one-pot reaction).
   (b) The compound of formula (I-A) or a salt thereof may be prepared by subjecting to hydrogenation reaction the compound of formula (I-D), prepared according to the previous method.
      The hydrogenation reaction is known, for example, it is carried out in an inert solvent [ethers (e.g. tetrahydrofuran, dioxane, dimethoxyethane, diethyl ether, etc.), alcohols (e.g. methanol, ethanol, etc.), benzenes (e.g. benzene, toluene, etc.), ketones (e.g. acetone, methyl ethyl ketone, etc.), nitriles (e.g. acetonitrile etc.), amides (e.g. dimethylformamide etc.), water, ethyl acetate, acetic acid or a mixture of two of them, etc.] in the presence of hydrogenating catalyst (e.g. palladium-carbon, palladium black, palladium, palladium hydroxide, nickel, Raney nickel, ruthenium chloride, etc.), in the presence or absence of an inorganic acid (e.g. hydrochloric acid, sulfuric acid, hypochlorous acid, boric acid, tetrafluoroboric acid, etc.) or an organic acid (e.g. acetic acid, p-toluenesulfonic acid, oxalic acid, trifluoroacetic acid, formic acid, etc.), under the atmosphere of hydrogen under normal or suppressed pressure or in the presence of ammonium formate at a temperature of 0~200°C. When an acid is used, its salt may be applied instead.
   (c) The compound of formula (I-A) may be prepared by subjecting to a deprotection reaction 5-benzyloxycarbonylamino-6-oxo-1,6-dihydropyrimidin-1-ylacetic acid derivative of formula (V) 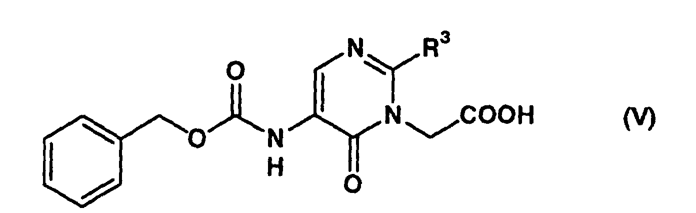 (wherein R³ has the same meaning as above described) or a salt thereof.
      The deprotection reaction is known, and it may be carried out according to the same method as above hydrogenation reaction.
   (d) The compound of formula (I-A) may be prepared by subjecting to reduction reaction 5-nitro-6-oxo-1,6-dihydropyrimidin-1-ylacetic acid derivative of formula (VI) 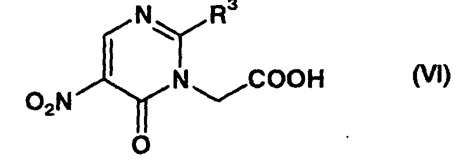 (wherein R³ has the same meaning as above described) or a salt thereof.

   Reduction reaction of nitro group is known, for example, it is carried out by hydrogenation reaction and reduction reaction using a metal or a salt thereof.
   Hydrogenation reaction may be carried out by the above method.
   The reaction using a metal or a salt thereof is known, for example, it is carried out in a water-miscible solvent (ethanol, methanol, etc.) in the presence or absence of hydrochloric acid, using a metal or a salt thereof (e.g. zinc, steel, tin, tin chloride, iron chloride, etc.) at a temperature of 50~150°C.
[5] (RS-N-[(1-(5-tert-butyl-1,3,4-oxadiazol-2-ylcarbonyl)-2-methylpropyl)-2-(5-amino-6-oxo-1,6-dihydropyrimidin-1-yl)acetamide derivatibe or a non-toxic salt thereof of formula (E) 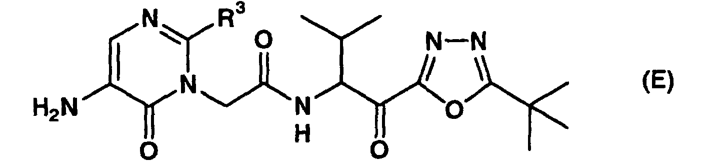 (wherein R³ has the same meaning as above described) or a non-toxic salt thereof may be prepared by subjecting to an amidation reaction the compound of formula (I-A), prepared according to the above method, or a non-toxic salt thereof and (RS)-2-(2-amino-3-methylbutyryl)-5-tert-butyl-1,3,4-oxadiazole or a salt thereof.

Amidation reaction is known, for example,
1) a method using acid halide,
2) a method using mixed anhydride,
3) a method using a condensing agent (EDC, DCC, etc.), etc. To explain these methods concretely,
   1) the method using acid halide is, for example, carried out by subjecting to a reaction the compound of formula (I-A) in an organic solvent (e.g. chloroform, methylene chloride, diethyl ether, tetrahydrofuran, etc.) or without a solvent, and acid-halide (e.g. oxalyl chloride, thionyl chloride, etc.) at a temperature of -20°C-refluxing temperature, and then subjecting to a reaction thus obtained acid halide in the presence of tertiary amine (e.g. pyridine, triethylamine, dimethylaniline, dimethylaminopyridine, etc.) at a temperature of -20~40°C.
   2) The method using mixed anhydride is, for example, carried out by subjecting to a reaction the compound of formula (I-A) in an organic solvent (e.g. chloroform, methylene chloride, diethyl ether, tetrahydrofuran, etc.) or without a solvent, in the presence of a tertiary amine (e.g. pyridine, triethylamine, dimethylaniline, dimethylaminopyridine, N-methylmorpholine, etc.) with acid halide (e.g. pivaloyl chloride, tosyl chloride, mesylchloride, etc.) or acid derivative (e.g. chloroethyl formate (chloroethyl carbonate), chloroisobutyl formate (chloroisobutyl carbonate), etc.) at a temperature of -20~40°C, and then subjecting to a reaction thus obtained mixed anhydride with the compound of formula (VII) in an inert organic solvent (chloroform, methylene chloride, diethyl ether, tetrahydrofuran, etc.) at a temperature of -20~40°C.
   3) The method using a condensing agent is, for example, carried out by subjecting to a reaction the compound of formula (I-A) and the compound of formula (VII) in an inert organic solvent (e.g. chloroform, methylene chloride, dimethylformamide, diethyl ether, tetrahydrofuran, ethyl acetate, pyridine, dimethylcarbonate, tert-butyl methyl ether, etc.) or without a solvent, in the presence or absence of an amine (e.g. pyridine, triethylamine, dimethylaniline, dimethylaminopyridine, sodium bicarbonate, etc.), using a condensing reagent (e.g. 1,3-dichlorohexylcarbodiimide (D_{C}C), 1-ethyl-3-[3-(dimethylamino)propyl ]carbodiimide (EDC), 1,1'-carbonyldiimidazole (CDI), 1,3-diisopropylcarbodiimide (DIPC), 2-chloro-1-methylpyridinium iodide, 1-benzotriazolylmesylate, etc.) in the presence or absence of 1-hydroxybenzotriazole (HOBt) at a temperature of -30~40°C.

The reactions of 1) and 2) are desirably carried out under atmosphere of inert gas (e.g. argon, nitrogen, etc.) under anhydrous conditions. The reaction of 3) may be carried out under atmosphere of inert gas (argon, nitrogen, etc.), both under anhydrous condition and in the presence of water.

The amidation reaction of the compound of formula (I-A) and the compound of formula (VII) is desirably carried out according to the reaction of 3) using a condensing agent.

The compounds of formula (II-1), ( II-2), (III), (IV-1), (IV-2), (V) or (VI) are known, for example, the compound of formula (II-1) is known as CAS registry No. 15646-46-5, 60777-96-0, the compound of formula (11-2) is known as CAS registry No. 171616-90-3, the compound among the compound of formula (III) wherein R³ is phenyl is known as CAS registry No. 32683-07-1, the compound of formula (IV-1) is known as CAS registry No. 3005-66-1, the compound among the compound of formula (V) and R³ is phenyl is known as CAS registry No. 148747-59-5 and the compound among the compound of formula (VI) wherein R³ is phenyl is described specifically in wool/23361 and the compound of formula (VII) in WOOO/55145.

Products of each reaction may be subjected to the next reaction after subjected to isolation, washing, drying and purification after each step or without subjecting to such operation. Otherwise, such operation may be ceased at an appropriate stage and go to the next scheme. Reaction products of each reaction may be purified by conventional purification techniques, e.g. distillation under normal or reduced pressure, high-performance liquid chromatography, thin layer chromatography, column chromatography, washing, recrystallization, etc.

The salts of the present invention include salts of alkali metals, salts of alkaline-earth metals, ammonium salts, salts of organic amine, acid-addition salts, etc.

The compounds of the present invention are converted into salts by known methods.

Appropriate salts include, salts of alkali metals (potassium, sodium, etc.), salts of alkaline-earth metals (calcium, magnesium, etc.), ammonium salts, salts of organic amines (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arguinine, N-methyl-D-glucamine, etc.), acid-addition salts (salts of inorganic acid such as hydrochloride, hydrobromide, sulfate, phosphate, nitrate, or acetate, trifluoroacetate, lactate, tartrate, oxalate, fumarate, maleate, citrate, benzoate, methanesulfonate, ethanesulfonate, benzenesulfonate, toluenesulfonate, isethionate, glucuronate, gluconate, etc.).

Non-toxic salts of the compound of formula (E) of the present invention are non-toxic ones of above described salts of alkali metals, salts of alkaline-earth metals, ammonium salts, salts of organic amines, acid-adduct salts, solvates.

The compound of the present invention and salts thereof may be converted into solvates (of water, methanol, etc.) by conventional methods.

### Industrial applicability

The method of the present invention excels the method previously used in the preparation of the compound of formula (E), particularly (RS)-N-[(1-(5-tert-butyl-1,3,4-oxadiazol-2-ylcarbonyl)-2-methylpropyl)-2-(5-amino-6-oxo-2-phenyl-1,6-dihydropyrimidin-1-yl)acetamide of formula (E-1) or a salt thereof.

That is, the previously known method required deprotection reaction after amidation, because pyridine compound whose 5-position amino group is protected was used in the reaction. However, the method of the present invention has made it possible to prepare the compound of formula (E), particularly the compound of formula (E-1), using pyridine compound whose 5-position amino group is not protected (i.e. without deprotecting amino group). Furthermore, the previous method was possible in a dilute solution of 0.2 mol/L but the present invention is possible in high density of 1.0 mol/1, therefore it facilitated industrial mass synthesis.

And from the point of view of step numbers for the preparation of the compound of formula (E), particularly the compound of formula (E-1), the method of the present invention is far more excellent than the method previously used.

That is, the method of the present invention gives the compound of formula (E-1) in 2-4 steps from benzamidinoacetic acid of formula (III), whereas the previous method gives the compound of formula (E-1) in 8 steps from N-(2,2-dimethoxyethyl)benzamidine (the method of W098/24806) or 7-10 steps from N-(2,2-dimethoxyethyl)phenylamidine (the method of WOOO/55145).

From above described, the method of the present invention is more suitable for industrial mass synthesis than the method previously used.

### Detailed description of the figures

Fig. 1 shows a single crystal structural data of the compound of example 4(4).

Fig.2 shows a single crystal structural packing data of the compound of example 4(4).

### Best mode for carrying out the present invention

The following reference examples and example illustrate the present invention, but the present invention is not limited to them.

The solvents in parentheses show the eluting or developing solvents and the ratios of the solvents used are by volume in chromatographic separations or TLC.

The solvents in parentheses in NMR show the solvents used in measurement.

### Example 1

### 5-benzoylamino-6-oxo-2-phenyl-1, 6-dihydropyrimidin-1-ylacetic acid

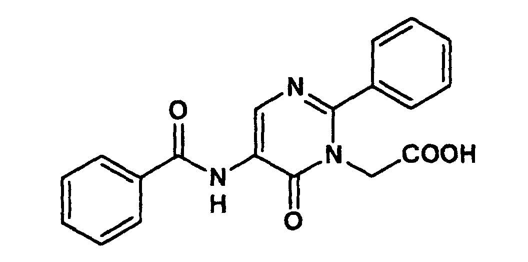

A solution of 20.9% sodium ethylate in ethanol (1.78 g) was diluted with anhydrous ethanol (8 ml) and thereto was added benzamidinoacetic acid (928 mg) under atmosphere of argon. The reaction mixture was stirred for 30 minutes at room temperature. To the reaction mixture was added 4-ethoxymethylen-2-phenyloxazolin-5-one (1.13 g). The reaction mixture was stirred for 150 minutes at room temperature and was refluxed for 2 hours. The reaction mixture was cooled to ambient temperature and thereto was added water (50 ml). To the mixture was added conc. hydrochloric acid to adjust pH 2 under cooling with ice. The reaction mixture was stirred for 1 hour at the same temperature. The precipitated solid substance was collected by filtration and was washed with water, dried at 55°C to give the compound of the present invention (1.58 g) having the following physical data.
TLC:Rf 0.27 (chloroform:methanol:acetic acid=18:1:0.8),
NMR (DMSO-d₆) :δ 13.33 (br s, 1H), 9.53 (s, 1H), 8.78 (s, 1H), 8.01-7.94 (m, 2H), 7.64-7.40 (m, 8H), 4.55 (s, 2H),
m.p.:244.0-245.3°C.

### Example 2

### 4-hydroxy-5-benzyloxycarbonylamino-6-oxo-2-phenyl-1,6-dihydropyrimidin-1-ylacetic acid

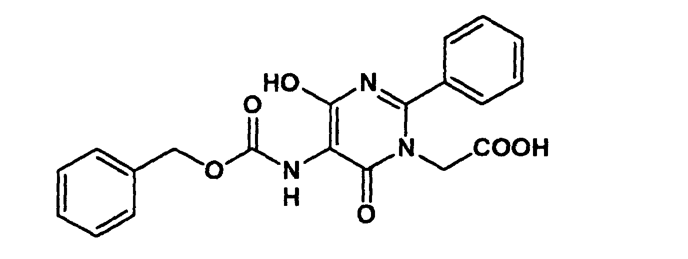

A mixture of phenol (21.2 g), dimethyl carbonate (23 ml) and 60% sodium hydride (4.5 g) was stirred for 30 minutes at 60°C. To the reaction mixture were added benzylamidino acetic acid (9.62 g) and dimethyl benzyloxycarbonyl aminomalonate (12.7 g) at 80°C. The reaction mixture was stirred for 8 hours at 80°C. The reaction mixture was cooled to ambient temperature and the reaction mixture was poured to 2N hydrochloric acid (70 ml) and was extracted with ethyl acetate. The extract was washed with an aqueous solution of sodium chloride, dried over anhydrous magnesium sulfate and was concentrated. To the residue was added toluene and was stirred to cause crystallization. The precipitated crystal was collected by filtration, dried to give the compound of the present invention (9.31 g) having the following physical data.
TLC:Rf 0.52 (chloroform:methanol:acetic acid=20:2:1),
NMR (DMSO-d₆): δ 8.30(brs, 1H), 7.53(s, 5H), 7.45-7.30 (m, 5H), 5.07(s, 2H), 4.43(s, 2H).

### Example 3

### 4-chloro-5-benzyloxycarbonylamino-6-oxo-2-phenyl-1,6-dihydropyrimidin-1-ylacetic acid

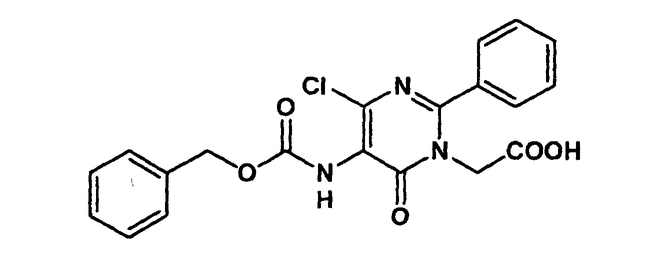

To a mixture of the compound prepared in example 2 (395 mg), N,N-dimethylaniline (328 mg) and dichloromethane (1 ml) was added phosphorous oxychloride (0.495 ml) dropwise at 45°C. The reaction mixture was stirred for 1 hour at 45°C. To the reaction mixture was added water and extracted with ethyl acetate (15 ml x 2). The extract was extracted with IN aqueous solution of sodium hydroxide. The aqueous layer was acidified with 6N hydrochloric acid and was extracted with ethyl acetate (15 ml x 2). The extract was washed with an aqueous solution of sodium chloride, dried over anhydrous magnesium sulfate and was concentrated. The residue was purified by column chromatography on silica gel (dichloromethane:methanol:acetic acid=30:1:1) to give the compound of the present invention (358 mg) having the following physical data.
TLC:Rf 0.41 (dichloromethane:methanol:acetic acid=20:1:1),
NMR (CDC1₃):δ 7.48(s, 5H), 7.40-7.28(m, 5H), 6.95(s, 1H), 5.17(s, 2H), 4.55(s, 2H).

### Example 4(1)

### 5-amino-6-oxo-2-phenyl-1,6-dihydropyrimidin-1-ylacetic acid sodium salt 2/3 methanol adduct

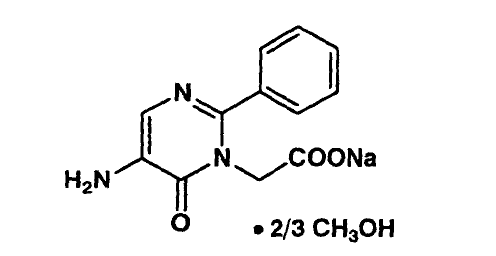

A solution of 28% sodium methylate in methanol (11 g) was diluted with methanol (57 ml) and to the mixture was added the compound prepared in example 1 (10 g) under atmosphere of argon. The reaction mixture was refluxed for 20 hours. The reaction mixture was cooled with ice and precipitated solid substance was collected by filtration and was washed with methanol, dried under reduced pressure at room temperature to give the compound of the present invention (7.33 g) having the following physical data.
TLC:Rf 0.64 (ethyl acetate:acetic acid:water=3:1:1),
NMR (DMSO-d₆):δ 7.54-7.48 (m, 2H), 7.44-7.30 (m, 3H), 7.26 (s, 1H), 4.94 (s, 2H), 4.14 (q, 1x2/3H, J=5.1 Hz), 4.03 (brs, 2H), 3.15 (d, 3X2/3H, J=5.1 Hz),
melting point:298-299°C.

| elementary analysis: C₁₂H₁₀N₃NaO₃ 2/3CH₄O | | | | | | |
|---|---|---|---|---|---|---|
| Calculated | C | 52.72% | H | 4.42% | N | 14.56 % |
| Found | C | 51.53% | H | 4.31% | N | 14.11 % |

### Example 4(2)

### 5-amino-6-oxo-2-phenyl-1,6-dihydropyrimidin-1-ylacetic acid sodium salt 2/3 methanol adduct

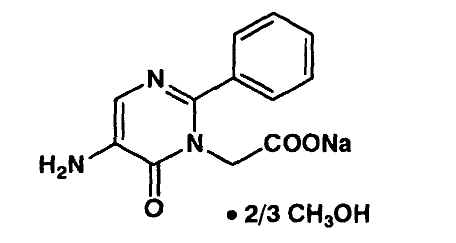

A solution of 20.9% sodium ethylate in ethanol (4.66 g) was diluted with anhydrous ethanol (24 ml) and thereto was added benzamidinoacetic acid (2.42 g) under atmosphere of argon. The reaction mixture was stirred for 30 minutes at room temperature. To the reaction mixture was added 4-ethoxymethylen-2-phenyloxazolin-5-one (2.95 g). The reaction mixture was stirred for 2 hours at room temperature and was refluxed for 90 minutes. The reaction mixture was cooled to ambient temperature. To the given reaction solution were added a solution of 28% sodium methylate in methanol (2.8 ml) and methanol (30 ml). The reaction mixture was refluxed for 16 hours. The reaction mixture was cooled with ice, and precipitated solid substance was collected by filtration, washed with methanol and was dried under reduced pressure at room temperature to give the compound of the present invention (3.08 g) having the same physical data the compound of example 4 (1).

### Example 4(3)

### 5-amino-6-oxo-2-phenyl-1,6-dihydropyrimidin-1-ylacetoc acid sodium salt

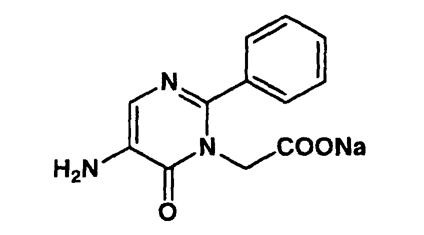

By drying the compound prepared in example 4(1) or 4(2) under reduced pressure at 80°C overnight, the compound of the present invention having the following physical data was given.
NMR (DMSO-d₆):δ 7.54-7.48 (m, 2H), 7.44-7.30 (m, 3H), 7.26 (s, 1H), 4.94 (s, 2H), 4.03 (brs, 2H),
melting point:295-296°C.

### Example 4(4)

### 5-amino-6-oxo-2-phenyl-1,6-dihydropyrimidin-1-ylacetic acid sodium salt methanol adduct

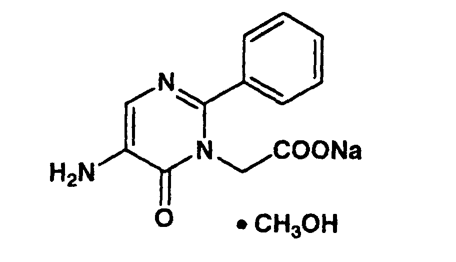

By recrystallizing the compound prepared in example 4(1) or 4(2) from methanol, the compound having the following physical data was given.
Single crystal X-ray diffraction spectrum structure analysis data

### [Measurement Condition]

Apparatus:manufacture of KK Rigaku, single crystal X-ray diffraction device AFC-5R
target:Cu,
filter:Ni filter,
Voltage:50 kV,
Current:200 mA,
Scanning speed:8.0° / min.

### [Result]

Crystallographic data were as shown below. lattice constant:a=14.05Å, b=6.60Å, c=27.51Å, β=89.99° , Space group:P2₁/c, (Z=8)
R factor:T=0.125

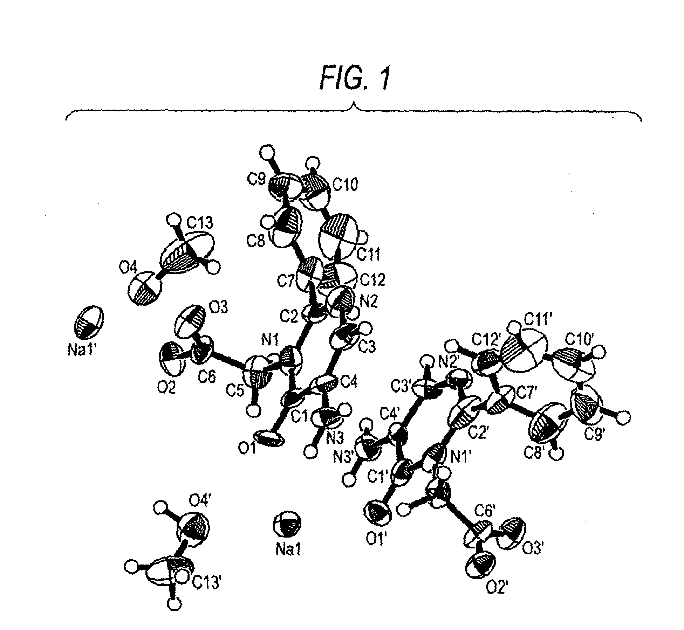

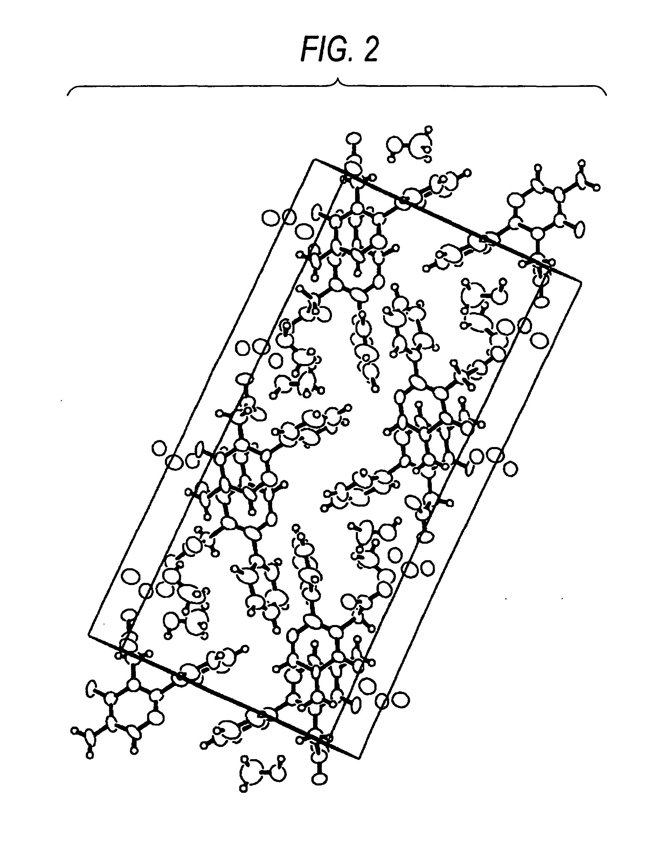

Single crystal structural data of the compound prepared in example 4(4) is shown in fig.1 and single crystal structural packing data are shown in fig.2.

### Example 4(5)

### 5-amino-6-oxo-2-phenyl-1,6-dihydropyrimidin-1-ylacetic acid

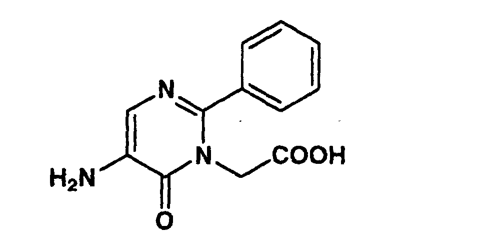

The compound prepared in example 4(1) or 4(2) (970 mg) was dissolved in water (10 ml) and to the mixture was added 2N hydrochloric acid (1.7 ml). The reaction mixture was cooled with ice, and thus given precipitate was filtered and was washed with ice and was dried under reduced pressure at 50°C to give the compound of the present invention (784 mg) having the following physical data.
TLC:Rf 0.64 (ethyl acetate:acetic acid:water=3:1:1),
NMR (DMSO-d₆):δ 13.05 (brs, 1H), 7.50-7.38 (m, 5H), 7.32 (s, 1H), 5.20 (brs, 2H), 4.45 (s, 2H),

| Elementary analysis: C₁₂H₁₁N₃O₃ | | | | | | |
|---|---|---|---|---|---|---|
| Calculated | C | 58.77% | H | 4.52% | N | 17.13% |
| Found | C | 58.60% | H | 4.39% | N | 16.98% |

### Example 5

### 5-amino-6-oxo-2-phenyl-1,6-dihydropyrimidin-1-ylacetic acid hydrochloride

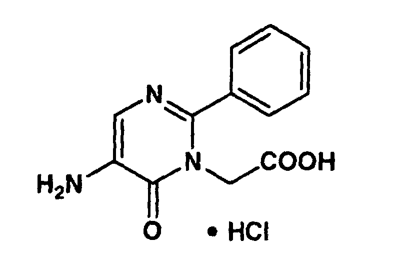

To a solution of the compound prepared in example 3 (30 mg) in methanol (5 ml) was added 10% palladium-carbon (50% wet, 10 mg). The reaction mixture was stirred for 2 hours at room temperature under atmosphere of hydrogen. The reaction mixture was collected by filtration and was concentrated. The given residue was dissolved in methanol (5 ml) and to the mixture was added 4N hydrochloric acid-ethyl acetate. The reaction mixture was concentrated to give the compound of the present invention (250 mg) having the following physical data.
TLC:Rf 0.55 (ethyl acetate:acetic acid:water=3:1:1),
NMR (CD₃OD):δ 7.80- 7.60 (m, 6H), 4.71 (s, 2H).

### Example 6(1)

### 5-amino-6-oxo-2-phenyl-1,6-dihydropyrimidin-1-ylacetic acid

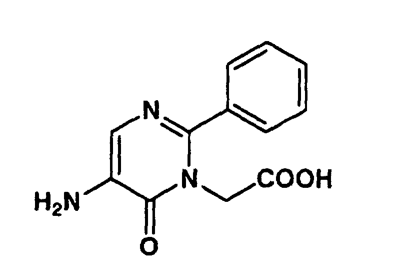

To a solution of 5-benzyloxycarbonylamino-6-oxo-2-phenyl-1,6-dihydropyrimidin-1-ylacetic acid (1.77 kg) in methanol (26 L) was added 10% palladium-carbon (50% wet, 70.8 g). The reaction mixture was stirred for 2 hours at room temperature under the atmosphere of hydrogen gas of 0.2 MPa pressure. The reaction mixture was filtrated and was concentrated. The residue was recrystallized from methanol to give the compound of the present invention (864 g) having the same physical data as the compound of example 4(5).

### Example 6(2)

### 5-amino-6-oxo-2-phenyl-1,6-dihydropyrimidin-1-ylacetic acid

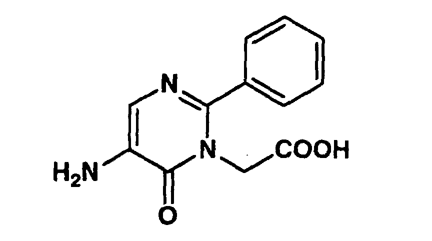

To a solution of 5-nitro-6-oxo-2-phenyl-1,6-dihydropyrimidin-1-ylacetic acid (275 mg) in methanol (2 ml) and tetrahydrofuran (2 ml) was added 10% palladium-carbon (50% wet, 100 mg). The reaction mixture was stirred for 5 hours at room temperature under the atmosphere of hydrogen. The reaction mixture was filtrated and was concentrated. The residue was recrystallized from methanol:toluene=1:5 to give the compound of the present invention (139 mg) having the same physical data as the compound of example 4(5).

### Example 7(1)

### (RS)-N-[1-(5-(tert-butyl-1,3,4-oxadiazol-2-ylcarbonyl)-2-methylpropyl)-2-(6-oxo-2-phenyl-5-amino-1,6-dihydropyrimidin-1-yl)acetamide

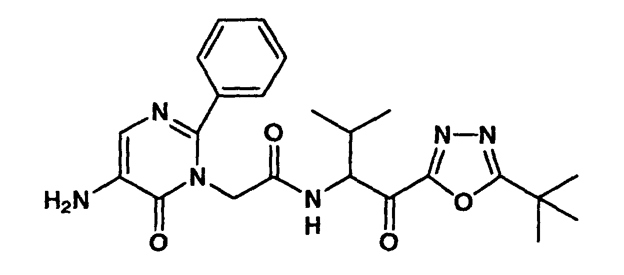

To a suspension of 1-hydroxybenzotriazole monohydrate (1.68 g) in acetonitrile (10 ml) were added (RS)-2-(2-amino-3-methylbutyryl)-5-tert-butyl-1,3,4-oxadiazole hydrochloride (2.88 g), 1-ethyl-3-(3-dimethylaminopropyl) carbodiimide hydrochloride (2.11 g) and the compound prepared in example 4(1) or 4(2) (2.87 g) successively. The reaction mixture was stirred for 3.5 hours at the same temperature. To the reaction mixture were added t-butyl methyl ether (40 ml) and water (20 ml) and the mixture was stirred for 30 minutes under cooling with ice. The precipitated solid substance was collected by filtration and washed with water (twice) and t-butyl methyl ether and was dried under reduced pressure at 60°C to give a crude product (2.82 g). This crude product was dissolved in methanol (79 ml) at 30°C. This solution was filtered and to the filtrate was added water (84 ml). The given solution was stirred for 5 hours at room temperature and for 1 hour under cooling with ice. The precipitated crystal was filtered and was washed with water and was dried under reduced pressure at 60°C to give the title compound (2.56 g) having the following physical data.
TLC:Rf 0.60 (dichloromethane:ethyl acetate:ethanol=10:10: 1),
NMR (CDCl₃) :δ 7.60-7.35 (m, 6H) , 6.92 (d, J=8.2Hz, 1H), 5.44 (dd, J=8.2, 4.9Hz, 1H), 4.68 (d, J=15.4Hz, 1H), 4.58 (d, J=15.4Hz, 1H), 2.64-2.40 (m, 1H), 1.48 (s, 9H), 1.07 (d, J=6.8Hz, 3H), 0.88 (d, J=6.8Hz, 3H).

### Example 7(2)

### (RS)-N-[1-(5-tert-butyl-1,3,4-oxadiazol-2-ylcarbonyl)-2-methylpropyl] 1-2-(6-oxo-2-phenyl-5-amino-1,6-dihydropyrimidin-1-yl)acetamide

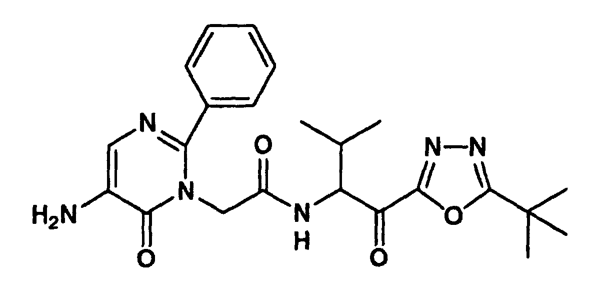

To a suspension of the compound prepared in example 4(1) or 4(2) (1.44 g) and 1-hydroxybenzotriazole monohydrate (919 mg) in dimethylformamide (3 ml) was added mesyl chloride (0.425 ml) at -10°C dropwise. The reaction mixture was stirred at the same temperature for 30 minutes. To the reaction mixture was added a solution of (RS)-2-(2-amino-3-methylbutyryl)-5-tert-butyl-1,3,4-oxadiazole hydrochloride (1.44 g) in dimethylformamide (2 ml). To the reaction mixture was added triethylamine (1.67 ml) at -10°C. The reaction mixture was stirred for 2 hours at -10~-5°C and for 1 hour at -5~0°C. To the reaction mixture was added water (50 ml) and was extracted with ethyl acetate:toluene=1:1 (twice). The extract was washed with 10% aqueous solution of citric acid, a saturated aqueous solution of sodium bicarbonate and a saturated aqueous solution of sodium chloride and dried over anhydrous magnesium sulfate and was concentrated. The given residue was purified by column chromatography on silica gel (ethyl acetate:toluene=3:1) to give the title compound (1.67 g) having the same physical data as the compound of example 7(1).

### Example 7(3)

### (RS)-N-[1-(5-tert-butyl-1,3,4-oxadiazol-2-ylcarbonyl)-2-methylpropyl]-2-(6-oxo-2-phenyl-5-amino-1,6-dihydropyrimidin-1-yl)acetamide

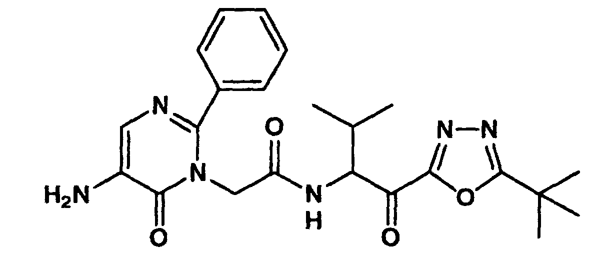

To a solution of the compound prepared in example 4(5) 6(1) or 6(2) (1.23 g) and 1-benzotriazolyl mesylate (1.28 g) on dimethylformamide (3 ml) was added a solution of (RS)-2-(2-amino-3-methylbutyryl)-5-tert-butyl-1,3,4-oxadiazole hydrochloride (1.44 g) in dimethylformamide (2 ml). To the reaction mixture was added triethylamine (1.67 ml) at -5°C dropwise. The reaction mixture was stirred for 2 hours at - 5~0°C. To the reaction mixture was added water and was extracted with ethyl acetate:toluene=1:1. The extract was washed with 10% aqueous solution of citric acid, water, a saturated aqueous solution of sodium bicarbonate, water and a saturated aqueous solution of sodium chloride and dried over anhydrous magnesium sulfate and was concentrated. The residue was purified by column chromatography on silica gel (ethyl acetate:toluene=3:1) to give the title compound (1.73 g) having the same physical data as the compound of example 7(1).

### Example 7(4)

### (RS)-N-[1-(5-tert-butyl-1,3,4-oxadiazol-2-ylcarbonyl)-2-methylpropyl]-2-(6-oxo-2-phenyl-5-amino-1,6-dihydropyrimidin-1-yl)acetamide

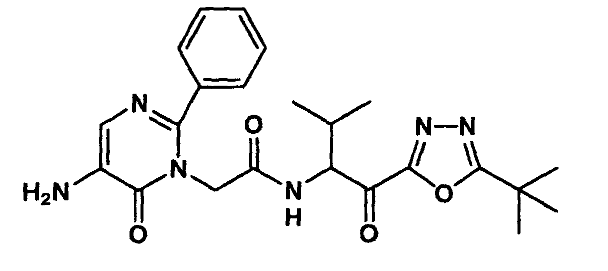

To a solution of the compound prepared in example 4(5), 6(1) or 6(2) (490 mg) and 1-benzotriazolylmesylate (426 mg) in dimethylformamide (8 ml) was added triethylamine at 0°C. The reaction mixture was stirred for 1 hour at the same temperature. To the reaction mixture was added a solution of (RS)-2-(2-amino-3-methylbutyryl)-5-tert-butyl-1,3,4-oxadiazole hydrochloride (628 mg) in dimethylformamide (2 ml). To the reaction mixture was added triethylamine (0.33 ml) at 0°C. The reaction mixture was stirred for 1 hour at 0°C. To the reaction mixture was added water and was extracted with ethyl acetate:toluene=1:1. The extract was washed with 10% aqueous solution of citric acid, water, a saturated aqueous solution of sodium bicarbonate, water, a saturated aqueous solution of sodium chloride and dried over anhydrous magnesium sulfate and was concentrated. The residue was purified by column chromatography on silica gel (ethyl acetate:toluene=3:1) to give the title compound (756 mg) having the same physical data as the compound of example 7(1).

### Example 8

### 5-benzoylamino-6-oxo-2-(4-fluorophenyl)-1,6-dihydropyrimidin-1-ylacetic acid

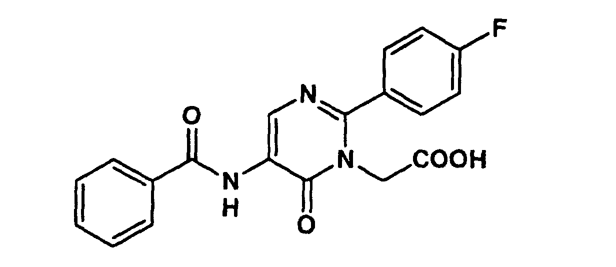

A solution of 20.9% sodium ethylate in ethanol (9 g) was diluted by anhydrous ethanol (40 ml) and under atmosphere of argon thereto was added 4-fluorobenzamidinoacetic acid (4.9 g). The reaction mixture was stirred for 30 minutes at room temperature. To the reaction mixture was added 4-ethoxymethelen-2-phenyloxazolin-5-one (5.79 g). The reaction mixture was stirred for 30 minutes at room temperature and was refluxed for 4 hours. The reaction mixture was cooled with ice and thereto was added IN hydrochloric acid (27.5 ml). The mixture was stirred for 1 hour at the same temperature. The precipitate was collected by filtration and washed with water and was dried under reduced pressure at 55°C to give the compound of the present invention (8.24 g) having the following physical data.
TLC:Rf 0.47 (chloroform:methanol:acetic acid:water=50:10:1:1),
NMR (DMSO-d₆):δ 9.53 (s, 1H), 8.78 (s, 1H), 7.97 (d, J=7.2 Hz, 2H,), 7.65-7.5 (m, 5H), 7.36 (t, J=8.8 Hz, 2H), 4.52 (s, 2H).

### Example 8(1)~8(18)

By the same procedure as described in example 8 using the corresponding amidinoacetic acid derivative in place of 4-fluorobenzamidinoacetic acid, the compound of the present invention having the following physical data was given.

### Example 8(1)

### 5-benzoylamino-6-oxo-2-(3-bromophenyl) -1,6-dihydropyrimidin-1-ylacetic acid

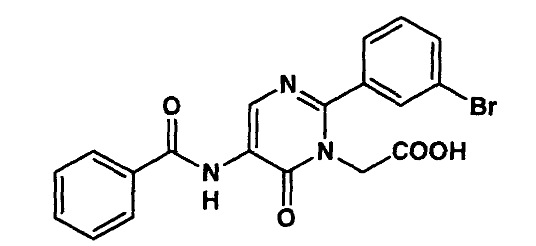

NMR (DMSO-d₆): δ 9.53 (s, 1H), 8.79 (s, 1H), 8.01 - 7.94 (m, 2H), 7.80 - 7.72 (m, 2H), 7.66 - 7.46 (m, 5H), 4.55 (s, 2H).

### Example 8(2)

### 5-benzoylamino-6-oxo-2-(4-bromophenyl)-1,6-dihydropyrimidin-1-ylacetic acid

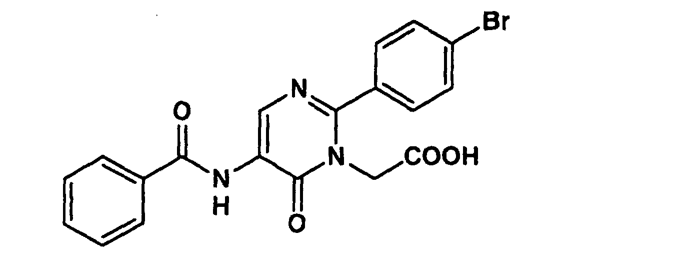

NMR (DMSO-d₆) :δ 9.53 (s, 1H), 8.79 (s, 1H), 7.97 (d, J=8.4 Hz, 2H), 7.75 (d, J=8.4 Hz, 2H), 7.66 - 7.46 (m, 5H), 4.56 (s, 2H).

### Example 8(3)

### 5-benzoylamino-6-oxo-2-(4-methoxyphenyl)-1,6-dihydropyrimidin-1-ylacetic acid

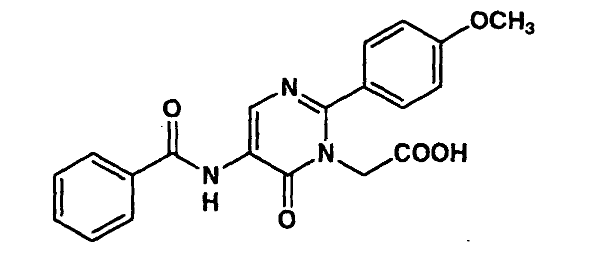

NMR (DMSO-d₆):δ 9.46 (s, 1H), 8.76 (s, 1H), 7.96 (d, J=8.7 Hz, 2H), 7.66 - 7.46 (m, 5H), 7.05 (d, J=8.7 Hz, 2H), 4.51 (s, 2H), 3.81 (s, 3H).

### Example 8(4)

### 5-benzoylamino-6-oxo-2-(4-nitrophenyl)-1,6-dihydropyrimidin-1-ylacetic acid

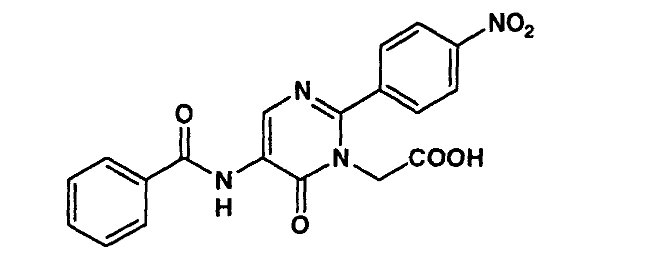

NMR (DMSO-d₆) :δ 9.59 (s, 1H), 8.83 (s, 1H), 8.38 (d, J=8.7 Hz, 2H), 7.97 (d, J=6.9 Hz, 2H), 7.82 (d, J=8.7 Hz, 2H), 7.65 - 7.50 (m, 3H), 4.57 (s, 2H).

### Example 8(5)

### 5-benzoylamino-6-oxo-2-(4-methylphenyl)-1,6-dihydropyrimidin-1-ylacetic acid

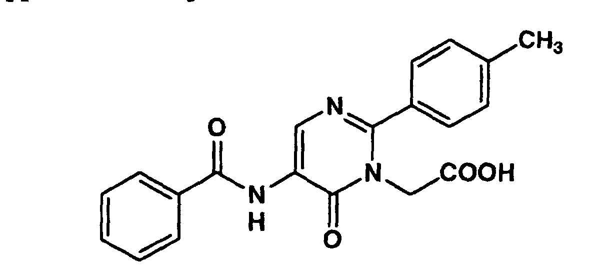

TLC:Rf 0.42 (ethyl acetate:methanol:acetic acid=90:10:1)
NMR (DMSO-d₆):δ 9.52(s, 1H), 8.77 (s, 1H), 7.97 (d, J=7.2 Hz, 2H), 7.62 (t, J=7.2 Hz, 1H), 7.54 (t, J=7.2 Hz, 2H),7.43 (d, J=8.4 Hz, 2H), 7.34 (d, J=8.4 Hz, 2H), 4.56 (s, 2H), 2.38 (s, 3H).

### Example 8(6)

### 5-benzoylamino-6-oxo-2-(3-trifluorophenyl)-1,6-dhydropyrimidin-1-ylacetic acid

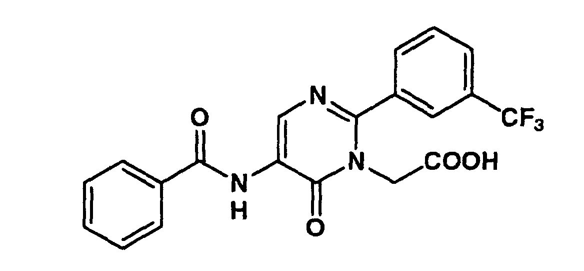

TLC:Rf 0.50 (ethyl acetate:methanol:acetic acid=90:10:1),
NMR (DMSO-d₆):δ 9.58(s, 1H), 8.82 (s, 1H), 8.01 - 7.75 and 7.66 - 7.51 (m, 9H), 4.57 (s, 2H).

### Example 8(7)

### 5-benzoylamino-6-oxo-2-(3-nitrophenyl)-1,6-dihydropyrimidin-1-ylacetic acid

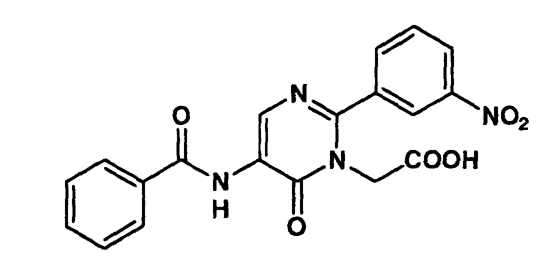

TLC:Rf 0.33 (ethyl acetate:methanol:acetic acid=90:10:1),
NMR (DMSO-d₆):δ 9.59 (s, 1H), 8.83 (s, 1H), 8.44 - 8.38 (m, 2H), 8.02- 7.84 (m, 3H), 7.88 - 7.80 (m 1H), 7.66 - 7.60 (m, 1H), 7.60 - 7.50 (m, 2H), 4.60 (s, 2H).

### Example 8(8)

### 5-benzoylamino-6-oxo-2-(4-benzyloxyphenyl)-1,6-dihydropyrimidin-1-ylacetic acid

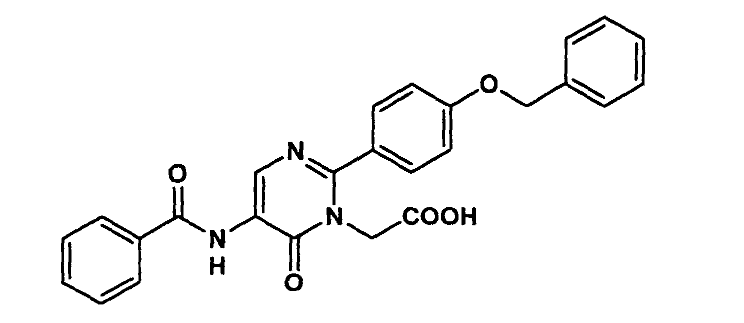

TLC:Rf 0.54 (ethyl acetate:methanol:acetic acid=90:10:1),
NMR (DMSO-d₆):δ 9.51 (s, 1H), 8.76 (s, 1H), 7.97 (d, J=6.9 Hz, 2H), 7.62 (t, J=6.9 Hz, 1H), 7.54 (t, J=6.9 Hz, 2H), 7.65 - 7.30 (m 7H), 7.16 (d, J=9.0 Hz, 2H), 5.18 (s, 2H), 4.59 (s, 2H).

### Example 8(9)

### 5-benzoylamino-6-oxo-2-(4-chlorophenyl)-1,6-dihydropyrimidin-1-ylacetic acid

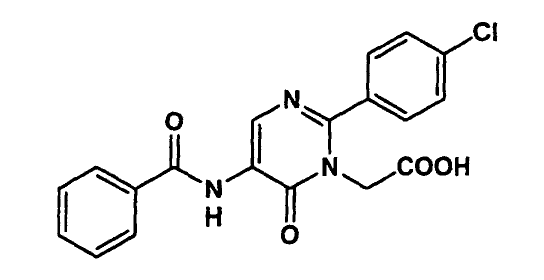

TLC:Rf 0.46 (ethyl acetate:methanol:acetic acid=8:2:1),
NMR (DMSO-d₆): δ 13.45-13.25 (m, 1H), 9.55 (s, 1H), 8.79 (s, 1H), 8.00-7.92 (m, 2H), 7.67-7.45 (m, 7H), 4.57 (s, 2H).

### Example 8(10)

### 5-benzoylamino-6-oxo-2-(4-trifluorophenyl ) -116-dihydropyrimidin-1-ylacetic acid

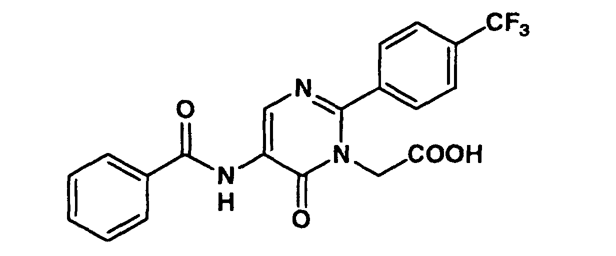

TLC:Rf 0.38 (ethyl acetate:methanol:acetic acid=8:2:1), NMR (DMSO-d₆):δ13.50-13.20 (m, 1H), 9.57 (s, 1H), 8.82 (s, 1H), 7.92 and 7.77 (each d, J=8.4 Hz, each 2H), 4.56 (s, 2H).

### Example 8(11)

### 5-benzoylamino-6-oxo-2-(3-benzyloxyphenyl)-1,6-dihydropyrimidin-1-ylacetic acid

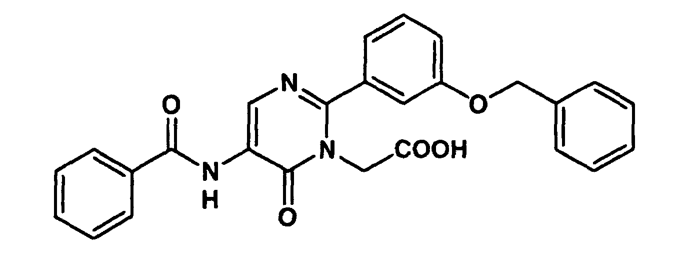

TLC:Rf 0.43 (ethyl acetate:methanol:acetic acid=8:2:1),
NMR (DMSO-d₆):δ 13.45-13.25 (m, 1H), 9.54 (s, 1H), 8.77 (s, 1H), 8.05-7.05 (m, 14H), 5.13 (s, 2H), 4.55 (s, 2H).

### Example 8(12)

### 5-benzoylamino-6-oxo-2-(2-methoxyphenyl)-1,6-dihydropyrimidin-1-ylacetic acid

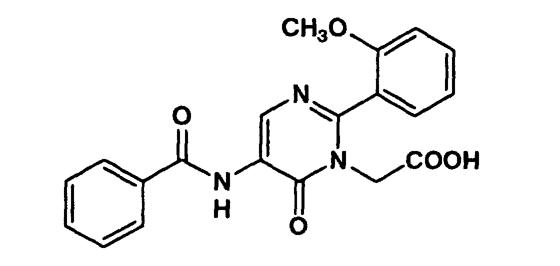

TLC:Rf 0.38 (chloroform:methanol:acetic acid=90:10:1),
NMR (DMSO-d₆):δ 13.15 (brs, 1H), 9.50 (s, 1H), 8.74 (s, 1H), 7.97-7.94 (m, 2H), 7.97-7.51 (m, 4H), 7.28-7.05 (m, 3H), 4.76 (d, J=17.1 Hz, 1H), 4.15 (d, J=17.1 Hz, 1H), 3.79 (s, 3H).

### Example 8 (13)

### 5-benzoylamino-6-oxo-2-(2,5-dimethoxyphenyl)-1,6-dihydropyrimidin-1-ylacetic acid

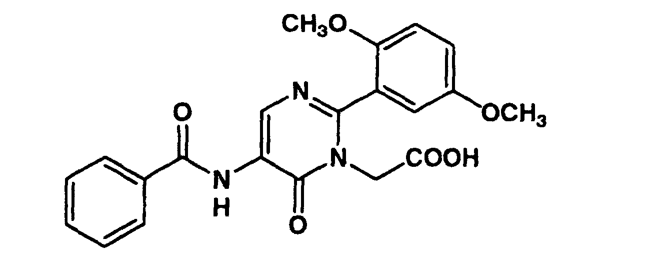

TLC:Rf 0.28 (chloroform:methanol:acetic acid=90:10:1),
NMR (DMSO-d₆):δ 13.20 (brs, 1H), 9.51 (s, 1H), 8.73 (s, 1H), .796 (d, J=7.2 Hz, 2H), 7.63-7.51 (m, 3H), 7.11 (s, 2H), 6.82 (s, 1H), 4.76 (d, J=17.1Hz, 1H), 4.16 (d, J=17.1 Hz, 1H), 3.73 (s, 3H), 3.71 (s, 3H).

### Example 8(14)

### 5-benzoylamino-6-oxo-2-(2,4-dimethoxyphenyl)-1,6-dihydropyrimidin-1-ylacetic acid

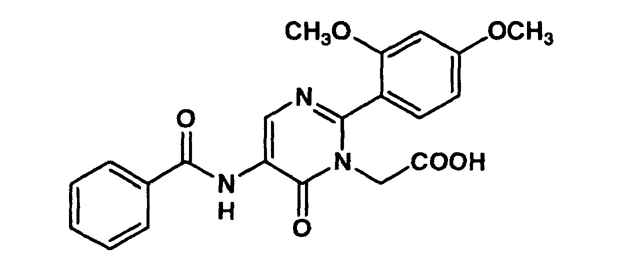

TLC:Rf 0.31 (chloroform:methanol:acetic acid=90 :10:1),
NMR (DMSO-d₆): δ 9.47(s, 1H), 8.72 (s, 1H), 7.96-7.93 (m, 2H), 7.61-7.50 (m, 3H), 7.20-7.17 (m, 1H), 6.69-6.62 (m, 2H), 4.75 (d, J=17.4 Hz, 1H), 4.18 (d, J=1.74 Hz, 1H), 3.82 (s, 3H), 3.78 (s, 3H).

### Example 8(15)

### 5-benzoylamino-6-oxo-2-methyl-1,6-dihydropyrimidin-1-ylacetic acid

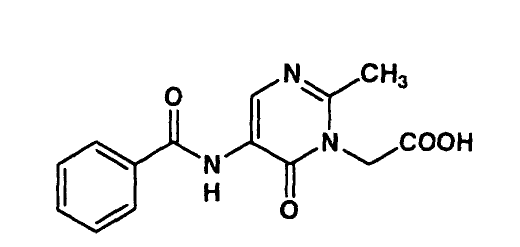

TLC:Rf 0.15 (chloroform:methanol:acetic acid:water=50:10:1:1),
NMR (DMSO-d₆):δ 9.39 (s, 1H), 8.54 (s, 1H), 7.93 (m, 2H), 7.65-7.50 (m, 3H), 4.13 (s, 2H), 2.46 (s, 3H).

### Example 8(16)

### 5-benzoylamino-6-oxo-2-benzyl-1,6-dihydropyrimidin-1-ylacetic acid

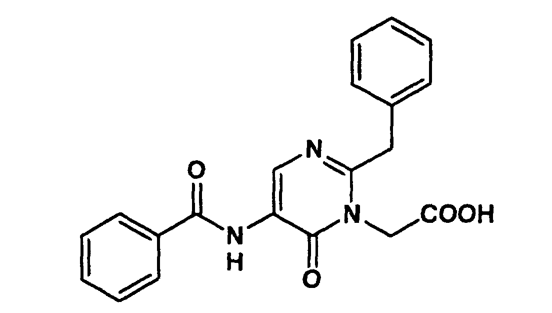

TLC:Rf 0.44 (chloroform:methanol:acetic acid:water=50:10: 1:1),
NMR (CDCl₃):δ 9.21 (s, 1H), 8.70 (s, 1H), 7.90 (d, J=6.9 Hz, 2H), 7.6-7.45 (m, 3H), 7.35-7.2 (m, 5H), 4.76 (s, 2H), 4.12 is, 2H).

### Example 8(17)

### 5-benzoylamino-6-oxo-2-(5-methylfuran-2-yl)-1,6-dihydropyrimidin-1-ylacetic acid

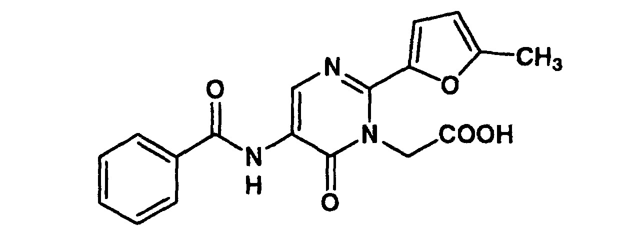

TLC:Rf 0.53 (ethyl acetate:methanol:acetic acid=90:10:1),
NMR (DMSO-d₆) :δ 9.45 (s, 1H), 8.75 (s, 1H), 7.81 (d, J=7.5 Hz, 2H), 7.61 (t, J=7.5 Hz, 1H), 7.53 (t, J=7.5 Hz, 2H), 7.19 (d, J=3.6 Hz, 1H), 6.39 (d, J=3.6 Hz, 1H), 5.05 (s, 2H), 2.35 (s, 3H).

### Example 8(18)

### 5-benzoylamino-6-oxo-2-(1,3-dioxaindan-5-yl)-1,6-dihydropyrimidin-1-ylacetic acid

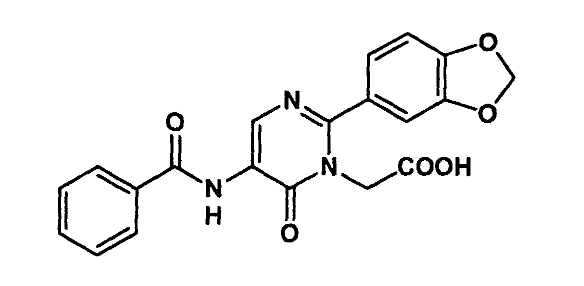

TLC:Rf 0.38 (ethyl acetate:methanol:acetic acid=90:10:1),
NMR (DMSO-d₆):δ 9.51 (s, 1H), 8.75 (s, 1H), 7.97 (d, J=6.9 Hz, 2H), 7.62 (t, J=6.9 Hz, 1H), 7.55 (t, J=6.9 Hz, 2H), 7.08 and 7.06 - 7.02 (each m, total 3H), 6.12 (s, 2H), 4.57 (s, 2H).

### Example 9

### 5-amino-6-oxo-2-(4-fluorophenyl)-1,6-dihydropyrimidin-1-ylacetic acid

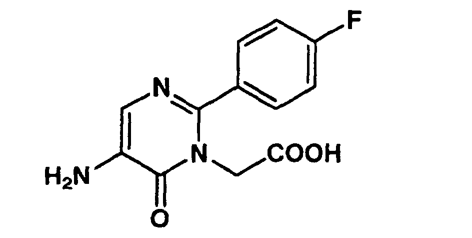

To a solution of 28% sodium methylate in methanol (3.86 g) in methanol (15 ml) was added the compound prepared in example 8 (3.67 g) under atmosphere of argon. The reaction mixture was refluxed for 20 hours. The reaction mixture was cooled with ice and precipitated solid substance was collected by filtration and was washed with methanol and dried under reduced pressure to give a sodium salt of the compound of the present invention (1.26 g). This sodium salt (1.19 g) was dissolved in water (4 ml) and thereto was added 1N hydrochloric acid (3.8 ml) and the mixture was stirred for 30 minutes. Thus precipitated solid substance was collected and dried to give the compound of the present invention (647 mg) having the following physical data.
TLC:Rf 0.08 (chloroform:methanol:acetic acid:water=50:10:10:1),
NMR (DMSO-d₆):δ 7.58 (dd, J=5.7, 9.0 Hz, 2H), 7.26 (s, 1H), 7.20 (t, J=9.0 Hz, 2H), 4.97 (s, 2H), 4.02 (br, 2H).

### Example 9(1)~9(18)

By the same procedure as described in example 9 using the compound prepared in example 8(1) ~ 8(18) in place of the compound prepared in example 8, the following compounds of the present invention were given.

### Example 9(1)

### 5-amino-6-oxo-2-(3-bromophenyl)-1,6-dihydropyrimidin-1-ylacetic acid

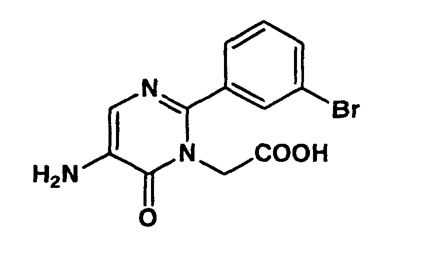

NMR (DMSO-d₆):δ 7.68 (m, 1H), 7.61 (m, 1H), 7.45 - 7.39 (m, 2H), 7.33 (s, 1H), 5.20 (br, 2H), 4.48 (s, 2H).

### Example 9(2)

### 5-amino-6-oxo-2-(4-bromophenyl)-1,6-dihydropyrimidin-1-ylacetic acid

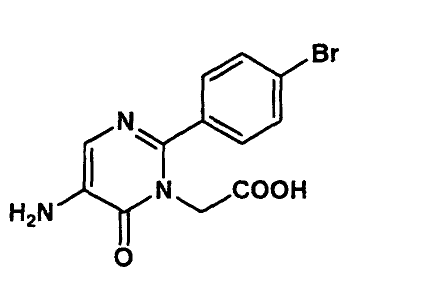

NMR (DMSO-d₆):δ 7.67 (d, J=8.7 Hz, 2H), 7.37 (d, J=8.7 Hz, 2H), 7.32 (s, 1H), 5.25 (br, 2H), 4.47 (s, 2H).

### Example 9(3)

### 5-amino-6-oxo-2-(4-methoxyphenyl ) -1 ,6-dihydropyrimidin-1-ylacetic acid

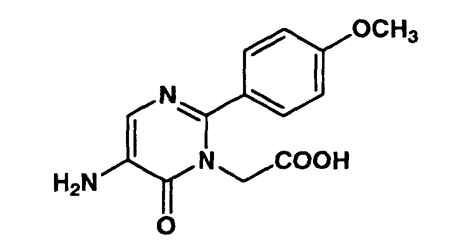

NMR (DMSO-d₆):δ 7.35 (d, J=9.0 Hz, 2H), 7.32 (s, 1H), 7.00 (d, J=9.0 Hz, 2H), 5.18 (br, 2H), 4.47 (s, 2H), 3.79 (s, 3H) .

### Example 9(4)

### 5-amino-6-oxo-2-(4-nitrophenyl)-1,6-dihydropyrimidin-1-ylacetic acid

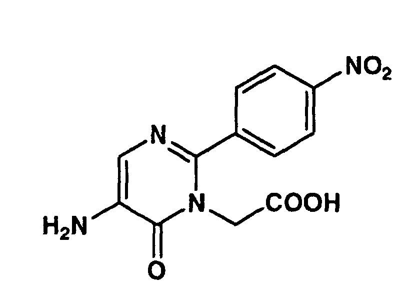

NMR (DMSO-d₆): δ 8.28 (d, J=8.7 Hz, 2H), 7.68 (d, J=8.7 Hz, 2H), 7.33 (s, 1H), 5.40 (brs, 2H), 4.47 (s, 2H).

### Example 9(5)

### 5-amino-6-oxo-2-(4-methylphenyl)-1,6-dihydropyrimidin-1-ylacetic acid

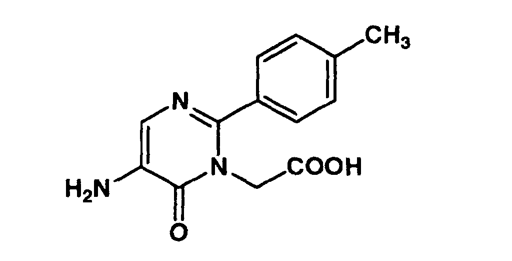

TLC:Rf 0.14 (ethyl acetate:methanol:acetic acid=90:10:1),
NMR (DMSO-d₆):δ 7.31 (s, 1H), 7.30 d, J=7.8 Hz, 2H), 7.25 (d, J=7.8 Hz, 2H), 5.15 (br s, 2H), 4.42(s, 2H), 2.34 (s, 3H).

### Example 9(6)

### 5-amino-6-oxo-2-(3-trifluorophenyl)-1,6-dihydropyrimidin-1-ylacetic acid

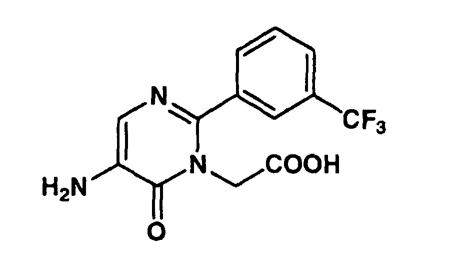

TLC:Rf 0.57 (ethyl acetate:methanol:acetic acid=18:1:1),
NMR (DMSO-d₆):δ 7.83 (d, J=8.4 Hz, 1H), 7.79 (s, 1H), 7.77 (d, J=8.4 Hz, 1H), 7.69 (t, J=8.4 Hz, 1H), 7.33 (s, 1H), 5.26 (br s, 2H), 4.37(s, 2H).

### Example 9(7)

### 5-amino-6-oxo-2-(3-nitrophenyl)-1,6-dihydropyrimidin-1-ylacetic acid

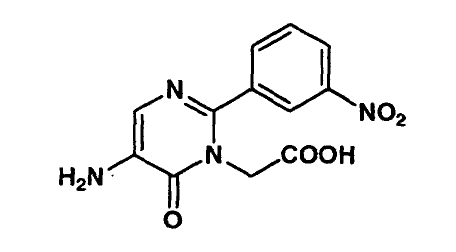

TLC:Rf 0.33 (ethyl acetate:methanol:acetic acid=18:1:1),
NMR (DMSO-d₆):δ 8.32 (d, J=7.8 Hz, 1H), 8.27 (s, 1H), 7.89 (d, J=7.8 Hz, 1H), 7.77 (t, J=7.8 Hz, 1H), 7.36 (s, 1H), 5.37 (brs, 2H), 4.51(s, 2H).

### Example 9(8)

### 5-amino-6-oxo-2-(4-benzyloxyphenyl)-1,6-dihydropyrimidin-1-ylacetic acid

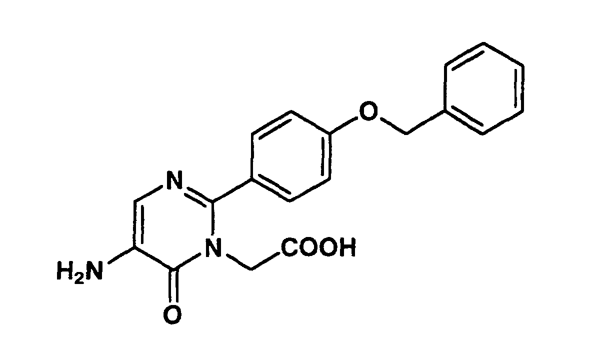

TLC:Rf 0.35 (ethyl acetate:methanol:acetic acid=8:1:1),
NMR (DMSO-d₆):δ 7.54 - 7.28 (m, 8H), 7.08 (d, J=8.7 Hz, 2H), 5.41 (s, 4H), 4.47 (s, 2H).

### Example 9(9)

### 5-amino-6-oxo-2-(4-chlorophenyl)-1,6-dihydropyrimidin-1-ylacetic acid

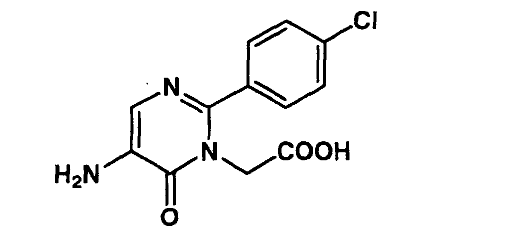

TLC:Rf 0.36 (ethyl acetate:methanol:acetic acid=6:3:1),
NMR (DMSO-d₆):δ 13.20-12.80 (m, 1H), 7.93 (d, J=8.4 Hz, 2H), 7.44 (d, J=8.4 Hz, 2H), 7.32 (s, 1H), 5.35-5.15 (m, 2H), 4.47 (s, 2H).

### Example 9(10)

### 5-amino-6-oxo-2-(4-trifluorophenyl)-1,6-dihydropyrimidin-1-ylacetic acid

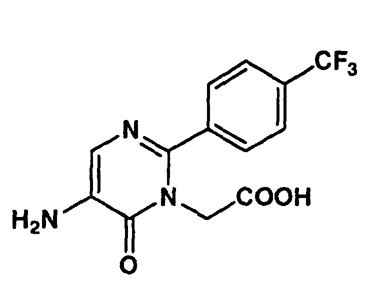

TLC:Rf 0.23 (ethyl acetate:methanol:acetic acid=8:2:1),
NMR (DMSO-d₆):δ 13.30-12.80 (m, 1H), 7.84 and 7.65 (each d, J=8.1 Hz, each 2H), 7.34 (s, 1H), 5.32 (brs, 2H), 4.47 (s, 2H).

### Example 9(11)

### 5-amino-6-oxo-2-(3-benzyloxyphenyl)-1,6-dihydropyrimidin-1-ylacetic acid

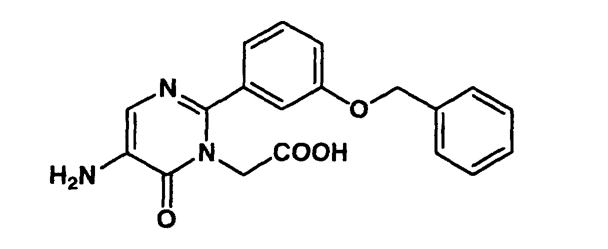

TLC:Rf 0.44 (ethyl acetate:methanol:acetic acid=6:3:1),
Mass (MALDI, pos.):352 (M + H)⁺.

### Example 9(12)

### 5-amino-6-oxo-2-(2-methoxyphenyl)-1,6-dihydropyrimidin-1-ylacetic acid

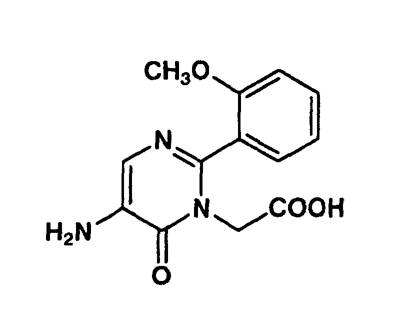

TLC:Rf 0.11 (chloroform:methanol:acetic acid=90:10:1),
NMR (CDCl₃:CD₃OD=7:1): δ 7.39-7.34 (m, 2H), 7.26-7.24 (m, 1H), 6.97-6.87 (m, 2H), 4.60 (d, J=16.5 Hz, 1H), 4.10 (d, J=16.5 Hz, 1H), 3.71 (s, 3H).

### Example 9(13)

### 5-amino-6-oxo-2- (2, 5-dimethoxyphenyl ) -1,6-dihydropyrimidin-1-ylacetic acid

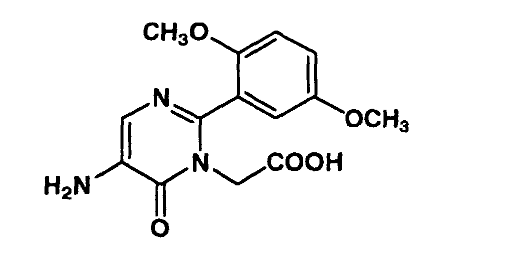

TLC:Rf 0.10 (chloroform:methanol:acetic acid=90:10:1),
NMR (CDCl₃:CD₃OD=7:1): δ 7.35 (s, 1H) , 6.88-6.76 (m, 3H), 4.94 (d, J=16.2 Hz, 1H), 4.01 (d, J=16. 2 Hz, 1H), 3.64 (s, 3H), 3.61 (s, 3H).

### Example 9(14)

### 5-amino-6-oxo-2-(2,4-dimethoxyphenyl)-1,6-dihydropyrimidin-1-ylacetic acid

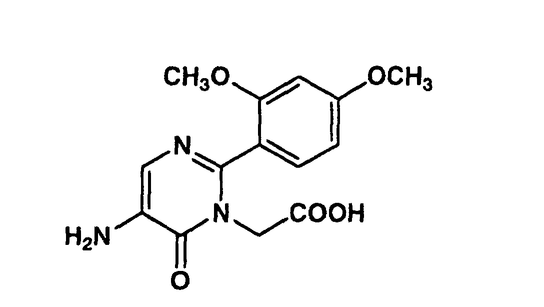

TLC:Rf 0.10 (chloroform:methanol:acetic acid=90:10:1),
NMR (CDCl₃:CD₃OD=7:1):δ 7.39 (s, 1H), 7.19-7.16 (m, 1H), 6.49-6.42 (m, 2H), 4.63 (d, J=16.8 Hz, 1H), 4.18 (d, J=16.8 Hz, 1H), 3.77 (s, 3H), 3.69 (s, 3H).

### Example 9(15)

### 5-amino-6-oxo-2-methyl-1,6-dihydropyrimidin-I-ylacetic acid

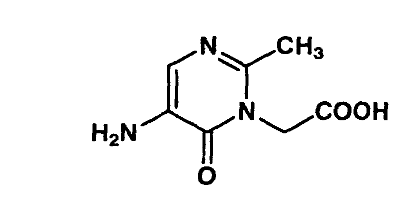

TLC:Rf 0.02 (chloroform:methanol:acetic acid:water=50:10:1:1),
NMR (DMSO-d₆):δ 7.14 (s, 1H), 4.85 (br, 2H), 4.72 (s, 2H), 2.27 (s, 3H).

### Example 9 (16)

### 5-amino-6-oxo-2-benzyl-1,6-dihydropyrimidin-1-ylacetic acid

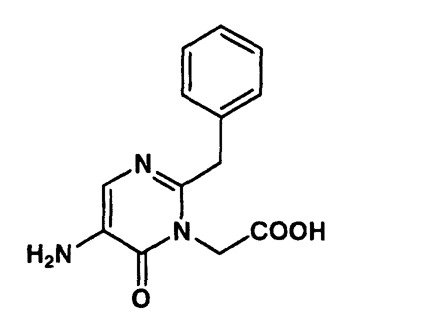

TLC:Rf 0.16 (chloroform:methanol:acetic acid:water=50:10:1:1) ,
NMR (CDCl₃) :δ 7.3-7.2 (m, 3H), 7.16 (s, 1H), 7.10 (d, J=7.5 Hz, 2H), 4.76 (s, 2H), 4.17 (br, 2H), 3.88 (s, 2H).

### Example 9(17)

### 5-amino-6-oxo-2-(5-methylfuran-2-yl)-1,6-dihydropyrimidin-1-ylacetic acid

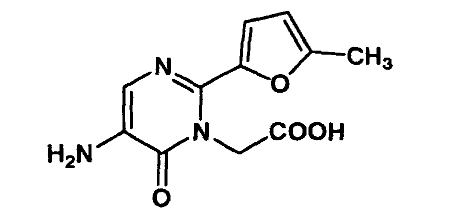

TLC:Rf 0.10 (ethyl acetate:methanol:acetic acid=90:10:1),
NMR (DMSO-d₆):δ 7.30 (s, 1H), 6.73 (d, J=3.3 Hz, 1H), 6.21 (d, J=3.3 Hz, 1H), 5.24 (brs, 2H), 4.75 (s, 2H), 2.28 (s, 3H).

### Example 9(18)

### 5-amino-6-oxo-2-(1,3-dioxaindan-5-yl)-1,6-dihydropyrimidin-1-ylacetic acid

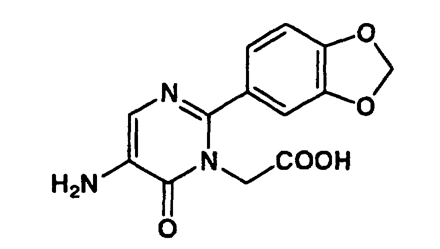

TLC:Rf 0.10 (ethyl acetate:methanol:acetic acid=90:10:1),
NMR (DMS0-d₆):δ 7.29(s, 1H), 6.99 (d, J=7.8 Hz, 1H), 6.94 (d,J=7.8 Hz, 1H), 6.89 (d, J=7.8 Hz, 1H), 6.08 (s, 2H), 5.16 (brs, 2H), 4.46(s, 2H).

## Claims

1. A pyrimidine derivative of formula (I) 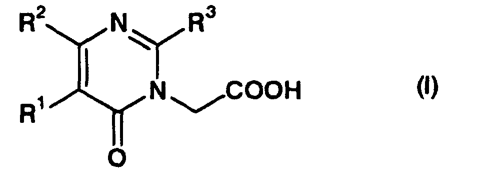 (wherein R¹ is amino, benzoylamino or benzyloxycarbonylamino, R² is hydrogen, hydroxy or chlorine,
R³ is (1) C1~4 alkyl,(2) Cycl or
(3) C1-4 alkyl substituted with Cycl
(wherein Cycl is C3~10 mono- or *bi-cyclic* carboring or 3~10 membered mono- or bi-cyclic heteroring comprising 1-4 of nitrogen, 1-2 of oxygen and/or 1-2 of sulfur and Cycl may be substituted with 1-5 of R⁴,
R⁴ is
(1) C1~4 alkyl, (2) halogen, (3) nitro, (4) trifluoromethyl, (5) trifluoromethoxy, (6) nitrile, (7) phenyl or (8) -OR⁵ (wherein R⁵ is C1-4 alkyl, phenyl, C1-4 alkyl substituted with phenyl)),
with proviso that when R¹ is benzyloxycarbonylamino, R² is hydroxy or chlorine) or a salt thereof.

2. The pyrimidine derivative compound according to claim 1, which is 5-amino-6-oxo-1,6-dihydropyrimidin-1-ylacetic acid derivative compound of formula (I-A) 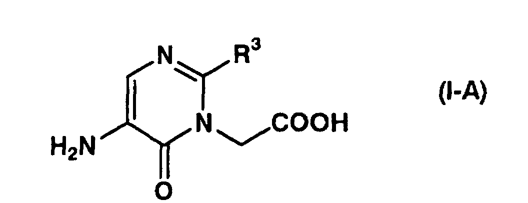 (wherein R³ has the same meaning as depicted in claim 1) or a salt thereof.

3. The pyrimidine derivative compound according to claim 1, which is 5-benzoylamino-6-oxo-1,6-dihydropyrimidin-1-ylacetic acid derivative of formula (I-B) 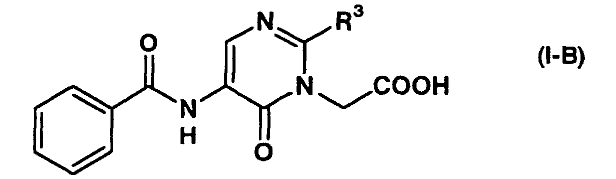 (wherein R³ is the same meaning as depicted in claim 1) or a salt thereof.

4. The pyrimidine derivative according to claim 1, which is 4-hydroxy-5-benzyloxycarbonylamino-6-oxo-1,6-dihydropyrimidin-1-ylacetic acid derivative compound of formula (I-C) 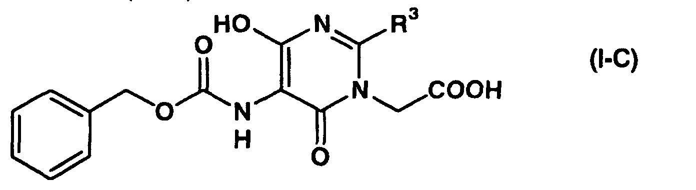 (wherein R³ is the same meaning as depicted in claim 1) or a salt thereof.

5. The pyrimidine derivative compound according to claim 1, which is 4-chloro-5-benzyloxycarbonylamino-6-oxo-1,6-dihydropyrimidin-1-ylacetic acid derivative compound of formula (I-D) 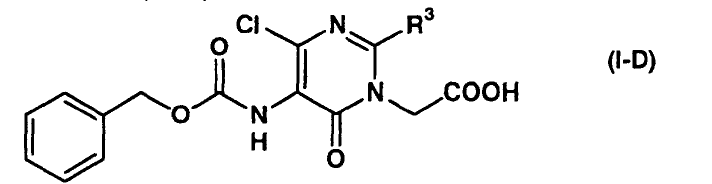 (wherein R³ is the same meaning as depicted in claim 1) or a salt thereof.

6. The pyrimidine derivative compound according to claim 2, which is
(1) 5-amino-6-oxo-2-phenyl-1,6-dihydropyrimidin-1-ylacetic acid,
(2) 5-amino-6-oxo-2-(4-fluorophenyl)-1,6-dihydropyrimidin-1-ylacetic acid,
(3) 5-amino-6-oxo-2-(3-bromophenyl)-1,6-dihydropyrimidin-1-ylacetic acid,
(4) 5-amino-6-oxo-2- (4-bromophenyl)-1,6-dihydropyrimidin-1-ylacetic acid,
(5) 5-amino-6-oxo-2-(4-methoxyphenyl)-1,6-dihydropyrimidin-1-ylacetic acid,
(6) 5-amino-6-oxo-2-(4-nitrophenyl)-1,6-dihydropyrimidin-1-ylacetic acid,
(7) 5-amino-6-oxo-2-(4-methylphenyl)-1,6-dihydropyrimidin-1-ylacetic acid,
(8) 5-amino-6-oxo-2-(3-trifluorophenyl)-1,6-dihydropyrimidin-1-ylacetic acid,
(9) 5-amino-6-oxo-2-(3-nitrophenyl)-1,6-dihydropyrimidin-1-ylacetic acid,
(10) 5-amino-6-oxo-2-(4-benzyloxyphenyl)-1,6-dihydropyrimidin-1-ylacetic acid,
(11) 5-amino-6-oxo-2-(4-chlorophenyl)-1,6-dihydropyrimidin-1-ylacetic acid,
(12) 5-amino-6-oxo-2-(4-trifluorophenyl)-1,6-dihydropyrimidin-1-ylacetic acid,
(13) 5-amino-6-oxo-2-(3-benzyloxyphenyl)-1,6-dihydropyrimidin-1-ylacetic acid,
(14) 5-amino-6-oxo-2-(2-methoxyphenyl)-1,6-dihydropyrimidin-1-ylacetic acid,
(15) 5-amino-6-oxo-2-(2,5-dimethoxyphenyl)-1,6-dihydropyrimidin-1-ylacetic acid,
(16) 5-amino-6-oxo-2-(2,4-dimethoxyphenyl)-1,6-dihydropyrimidin-1-ylacetic acid,
(17) 5-amino-6-oxo-2-methyl-1,6-dihydropyrimidin-1-ylacetic acid,
(18) 5-amino-6-oxo-2-benzyl-1 , 6-dihydropyrimidin-1 ylacetic acid,
(19) 5-amino-6-oxo-2-(5-methylfuran-2-yl)-1,6-dihydropyrimidin-1-ylacetic acid or
(20) 5-amino-6-oxo-2-(1,3-dioxaindan-5-yl)-1,6-dihydropyrimidin-1-ylacetic acid or a salt thereof.

7. The pyrimidine derivative compound according to claim 3, which is
(1) 5-benzoylamino-6-oxo-2-phenyl-1,6-dihydropyrimidin-1-ylacetic acid,
(2) 5-benzoylamino-6-oxo-2-(4-fluorophenyl)-1,6-dihydropyrimidin-1-ylacetic acid,
(3) 5-benzoylamino-6-oxo-2-(3-bromophenyl)-1,6-dihydropyrimidin-1-ylacetic acid,
(4) 5-benzoylamino-6-oxo-2-(4-bromophenyl)-1,6-dihydropyrimidin-1-ylacetic acid,
(5) 5-benzoylamino-6-oxo-2-(4-methoxyphenyl)-1,6-dihydropyrimidin-1-ylacetic acid,
(6) 5-benzoylamino-6-oxo-2-(4-nitrophenyl)-1,6-dihydropyrimidin-1-ylacetic acid,
(7) 5-benzoylamino-6-oxo-2-(4-methylphenyl)-1,6-dihydropyrimidin-1-ylacetic acid,
(8) 5-benzoylamino-6-oxo-2-(3-trifluorophenyl)-1,6-dihydropyrimidin-1-ylacetic acid,
(9) 5-benzoylamino-6-oxo-2-(3-nitrophenyl)-1,6-dihydropyrimidin-1-ylacetic acid,
(10) 5-benzoylamino-6-oxo-2-(4-benzyloxyphenyl)-1,6-dihydropyrimidin-1-ylacetic acid,
(11) 5-benzoylamino-6-oxo-2-(4-chlorophenyl)-1,6-dihydropyrimidin-1-ylacetic acid,
(12) 5-benzoylamino-6-oxo-2-(4-trifluorophenyl)-1,6-dihydropyrimidin-1-ylacetic acid,
(13) 5-benzoylamino-6-oxo-2-(3-benzyloxyphenyl)-1,6-dihydropyrimidin-1-ylacetic acid,
(14) 5-benzoylamino-6-oxo-2-( 2 -methoxyphenyl)-1,6 dihydropyrimidin-1-ylacetic acid,
(15) 5-benzoylamino-6-oxo-2-(2,5-dimethoxyphenyl)-1,6-dihydropyrimidin-1-ylacetic acid,
(16) 5-benzoylamino-6-oxo-2-(2,4-dimethoxyphenyl)-1,6-dihydropyrimidin-1-ylacetic acid,
(17) 5-benzoylamino-6-oxo-2-methyl-1,6-dihydropyrimidin-1-ylacetic acid,
(18) 5-benzoylamino-6-oxo-2-benzyl-1,6-dihydropyrimidin-1-ylacetic acid,
(19) 5-benzoylamino-6-oxo-2-(5-methylfuran-2-yl)-1,6-dihydropyrimidin-1-ylacetic acid or
(20) 5-benzoylamino-6-oxo-2-(1,3-dioxaindan-5-yl)-1,6-dihydropyrimidin-1-ylacetic acid or a salt thereof.

8. The pyrimidine derivative compound according to claim 4, which is
(1) 4-hydroxy-5-benzyloxycarbonylamino-6-oxo-2-phenyl-1,6-dihydropyrimidin-1-ylacetic acid or salt thereof.

9. The pyrimidine derivative compound according to claim 5, which is
(1) 4-chloro-5-benzyloxycarbonylamino-6-oxo-2-phenyl-1,6-dihydropyrimidin-1-ylacetic acid or a salt thereof.

10. A method for the preparation of the 5-benzoylamino-6-oxo-1,6-dihydropyrimidin-1-ylacetic acid derivative compound according to claim 3, **characterized by** subjecting to a reaction 4-ethoxyethylen-2-phenyloxazolin-5-one of formula (II-1) 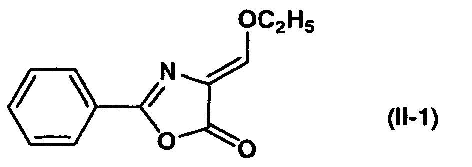 or 4-methoxymethylen-2-phenyloxazolin-5-one of formula (II-2) 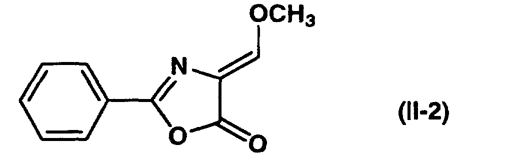 and an amidinoacetic acid derivative compound of formula (III) 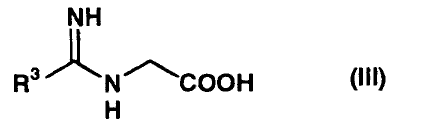 (wherein R³ has the same meaning as depicted in claim 1) or a salt thereof.

11. A method for the preparation of 5-amino-6-oxo-1,6-dihydropyrimidin-1-ylacetic acid derivative compound described in claim 2, **characterized by** subjecting to a deprotection reaction 5-benzoylamino-6-oxo-1,6-dihydropyrimidin-1-ylacetic acid derivative compound or a salt thereof described in claim 3.

12. A method for the preparation of 5-amino-6-oxo-1,6-dihydropyrimidin-1-ylacetic acid derivative compound described in claim 2 or a salt thereof, **characterized by** subjecting to a reaction 4-ethoxymethylen-2-phenyloxazolin-5-one of formula (II-1) 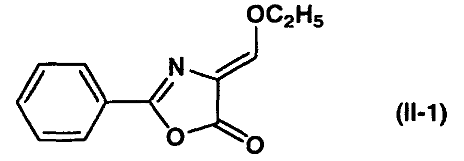 or 4-methoxymethylen-2-phenyloxazolin-5-one of formula (II-2) 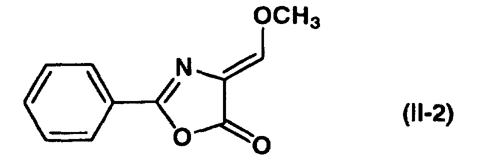 and an amidinoacetic acid derivative compound of formula fill) 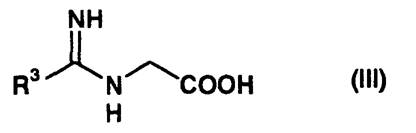 (wherein R³ has the same meaning as depicted in claim 1) or a salt thereof and followed by deprotection reaction.

13. A method for the preparation of 4-hydroxy-5-benzyloxycarbonylamino-6-oxo-1,6-dihydropyrimidin-1-ylacetic acid derivative compound described in claim 4, **characterized by** subjecting to a reaction the compound of formula (IV-1) 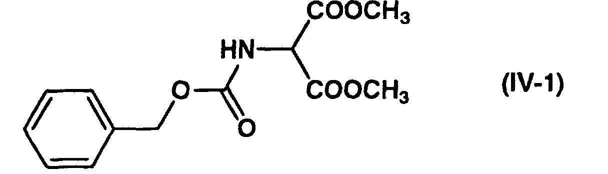 or dimethyl benzyloxycarbonylaminomalonate of formula (IV-2) 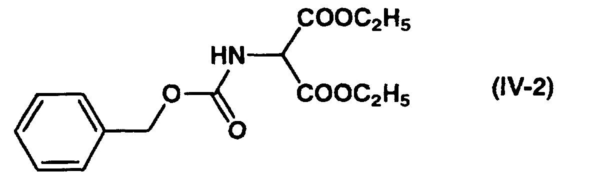 and an amidino acetic acid derivative compound of formula (III) 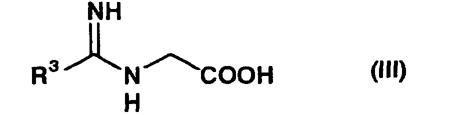 (wherein R³ has the same meaning as depicted in claim 1) or a salt thereof.

14. A method for the preparation of 4-chloro-5-benzyloxycarbonylamino-6-oxo-1,6-dihydropyrimidin-1-ylacetic acid derivative compound or a salt thereof, described in claim 5, **characterized by** subjecting to a halogenation reaction 4-hydroxy-5-benzyloxycarbonylamino-6-oxo-1,6-dihydropyrimidin-1-ylacetic acid derivative compound or a salt thereof described in claim 4.

15. A method for the preparation of 5-amino-6-oxo-1,6-dihydropyrimidin-1-ylacetic acid derivative compound or a salt described in claim 2, **characterized by** subjecting to a hydrogenation reaction 4-chloro-5-benzyloxycarbonylamino-6-oxo-1,6-dihydropyrimidin-1-ylacetic acid derivative compound or a salt thereof described in claim 5.

16. A method for the preparation of 5-amino-6-oxo-1,6-dihydropyrimidin-1-ylacetic acid derivative compound or a salt thereof according to claim 2, **characterized by** subjecting to a deprotection reaction 5-benzyloxy carbonylamino-6-oxo-1,6-dihydropyrimidin-1-ylacetic acid derivative compound of formula (V) 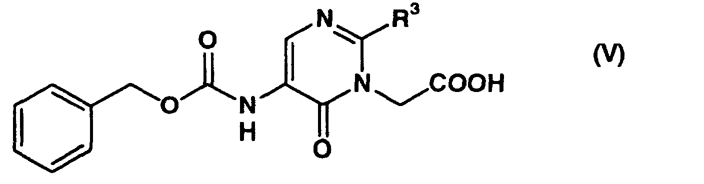 (wherein R³ has the same meaning as depicted in claim 1) or a salt thereof.

17. A method for the preparation of 5-amino-6-oxo-1,6 dihydropyrimidin-1-ylacetic acid derivative compound or a salt thereof according to claim 2, **characterized by** subjecting to a reduction reaction 5-nitro-6-oxo-1,6-dihydropyrimidin-1-ylacetic acid derivative compound of formula (VI) 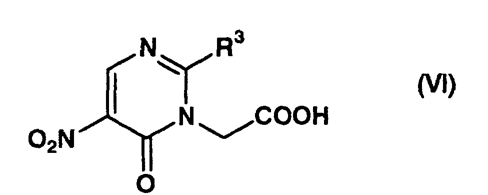 (wherein R³ has the same meaning as depicted in claim 1) or a salt thereof.

18. A method for the preparation of (RS)-N-[1-(5-tert-butyl-1,3,4-oxadiazol-2-ylcarbonyl)-2-methylpropyl]-2-(5-amino-6-oxo-1,6-dihydropyrimidin-1-yl)acetamide derivative of formula (E) 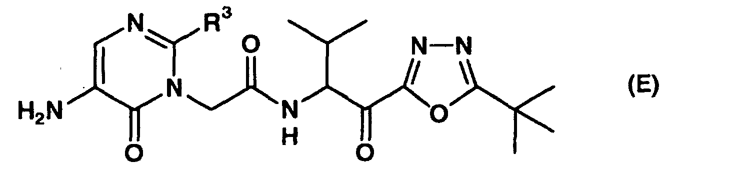 or a non-toxic salt thereof, **characterized by** subjecting to an amidation reaction 5-amino-6-oxo-1,6-dihydropyrimidin-1-ylacetic acid derivative compound according to claim 2 or a salt thereof and (RS)-2-(2-amino-3-methylbutyryl)-5-tert-butyl-1,3,4-oxadiazole of formula (VII) 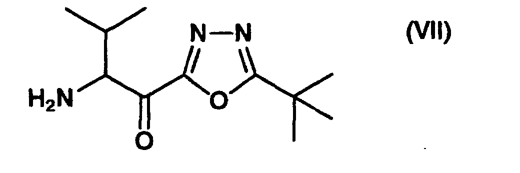 or a salt thereof.
